(19) Europäisches Patentamt
European Patent Office
Office européen des brevets

(11) EP 0 713 859 B1

(12) **EUROPEAN PATENT SPECIFICATION**

(45) Date of publication and mention of the grant of the patent: **30.08.2000 Bulletin 2000/35**

(21) Application number: **95118164.3**

(22) Date of filing: **17.11.1995**

(51) Int Cl.7: **C07C 219/10**, C07C 217/08, C07C 229/22, C07C 237/08, C07C 233/51, C07C 237/04, C07K 5/06, C07K 5/08, C08B 37/00, C08B 37/08

(54) **Cinnamic acid derivatives, photocrosslinkable cinnamic acid-polymer derivative and crosslinked cinnamic acid polymer derivatives**

Zimtsäure-Derivate, lichtvernetzbare Zimtsäure-Polymer-Derivate und vernetzte Zimtsäure-Polymer-Derivate

Dérivés d'acide cinnamique, dérivés polymères photoréticulables d'acide cinnamique et dérivés polymères réticulés d'acide cinnamique

(84) Designated Contracting States:
**AT BE CH DE DK ES FR GB GR IE IT LI LU MC NL PT SE**

(30) Priority: **17.11.1994 JP 30705094**
**20.09.1995 JP 26468695**

(43) Date of publication of application:
**29.05.1996 Bulletin 1996/22**

(73) Proprietor: **SEIKAGAKU KOGYO KABUSHIKI KAISHA (SEIKAGAKU CORPORATION)**
**Tokyo 103 (JP)**

(72) Inventors:
- **Waki, Michinori**
  **Higashimurayam-shi, Tokyo (JP)**
- **Miyamoto, Kenji**
  **Tachikawa-shi, Tokyo (JP)**
- **Motani, Yoshihiro**
  **Kobubunji-shi, Tokyo (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 284 632** **EP-A- 0 298 135**
**EP-A- 0 554 898**

- **MAKROMOL. CHEM. (1990), 191(12), 2985-91 , XP002001039 KLEMM, DIETER ET AL: "Photoreactive cellulose derivatives with comblike structure. 1. Modification of O-trialkylsilylcellulose with cinnamic acid-containing side chains"**
- **CHEMICAL ABSTRACTS, vol. 122, no. 15, 10 April 1995 Columbus, Ohio, US; abstract no. 188059, IBRAHIM, T.M. ET AL: "Synthesis of certain 4-nitrocinnamoylamino acids and dipeptide derivatives of biological interest" XP002001045 & EGYPT. J. PHARM. SCI. (1993), 34(1-3), 185-94 ,**
- **BULL. SOC. CHIM. BELG. (1976), 85(9), 647-56, XP002001040 DE POOTER, H. ET AL: "N-Acylamino acids and peptides. IV. The synthesis of N-cinnamyl-, N-p-coumaryl- and N-caffeylglycine and -glycyl-L-phenylalanine"**
- **J. AM. CHEM. SOC. (1990), 112(7), 2498-506, XP002001041 POPOVITZ-BIRO, R. ET AL: "A new series of amphiphilic molecules forming stable Z-type (polar) Langmuir-Blodgett films"**
- **J. CHROMATOGR. (1982), 234(1), 141-55 , XP002001042 VAN SUMERE, CHRISTIAAN F. ET AL: "Separation of some metabolically important aromatic N-acylamino acids of the benzoyl and cinnamoyl series by thin-layer, gas-liquid and high-performance liquid chromatography"**
- **J. CHROMATOGRAPHIE, vol. 20, no. 1, 1965, pages 48-60, XP002001043 C. F. VAN SUMERE ET AL.: "A new thin-layer metjod for phenolic substances and coumarins"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- JOURNAL OF THE AMERICAN CHEMICAL SOCIETY, vol. 80, no. 20, 1966, DC US, pages 4711-13, XP002001044 Y. SHALITIN ET AL.: "Cooperative effects of functional groups in peptides. II. Elimination reaction in aspartyl-(o-acyl)-serine derivatives"

## Description

This invention relates to a photodimerizable cinnamic acid derivative, a photocrosslinkable cinnamic acid-polymer derivative, and a crosslinked cinnamic acid-polymer derivative. The above cinnamic acid derivative and the cinnamic acid-polymer derivative, which is produced by introduction of said cinnamic acid derivative into a host polymer, are highly photoreactive compounds. Thus, on exposure to ultraviolet radiation, the cinnamic acid derivative undergoes an intermolecular crosslinking reaction (photodimerization reaction) to form a cyclobutane ring, thus enabling production of an insoluble crosslinked cinnamic acid-polymer derivative from said cinnamic acid-polymer derivative. Since a hyaluronic acid, biogenic substance, is selected as the host polymer, the resulting photocrosslinked cinnamic acid-polymer derivative can be a useful medical material having biocompatibility, bioresorptivity and other desirable characteristics.

### BACKGROUND OF THE INVENTION

Although a large number of photodimerizable compounds are known today, little information is available on the introduction of photocrosslinking groups into water-soluble host polymers. Technology is known in which photocrosslinked compounds were prepared by introducing various stilbazonium derivatives into the water-soluble polymer polyvinyl alcohol (JP-B-Sho-56-5762, JP-B-Sho-56-54155 and JP-B-Sho-61-12888, the term "JP-B" used herein means examined published Japanese patent application), but the objective of this technology is the immobilization of enzymes and bacteria and, moreover, the host polymers for introduction of photocrosslinking groups are chemically synthesized polymers. In these cases, in order to improve photoreactivity, the photodimerizable compounds are structurally modified to provide variations in absorption of ultraviolet region and its sensitivity. EP-A-0 554 898 or JP-A-Hei-6-73102 describes a technology in which cinnamic acid is introduced into the natural polymer glycosaminoglycan and the resulting derivative is crosslinked by means of ultraviolet irradiation (the term "JP-A" used herein means unexamined published Japanese patent application). Almost all of the examples given are directed to the preparation of a photocrosslinkable glycosaminoglycan compound through the direct introduction of cinnamic acid by way of ester linkage to the hydroxyl group of the host glycosaminoglycan, without the use of a spacer group, and the subsequent exposure of the compound to ultraviolet irradiation to provide a photocrosslinked product. This technology has the disadvantage that cinnamic acid must be introduced at a very high degree of substitution [DS (%): degree of substitution = 100 x (the number of moles of cinnamic acid introduced per constituent disaccharide unit] in order to carry out photocrosslinking to a sufficient extent and moreover that the efficiency of the photoreaction is poor. For example, DS necessary for induction of photocrosslinking reaction of a hyaluronic acid derivative incorporated with such photoreactive group (photodimerizable-crosslinkable group) as cinnamoyl group is fairly high (10% or more). In many of said hyaluronic acid derivative, intrinsic properties such as biodegradability, biocompatibility, non-toxicity, non-antigenicity, high swelling, etc. have been lost.

To cause crosslinking reaction of a hyaluronic acid derivative with low DS, it is favorable to use a hyaluronic acid with higher molecular weight. In this case, however, DS of cinnamoyl group is high and thus severe reaction conditions are required in accordance with the method developed previously. It is known that a hyaluronic acid is easily degraded to low molecule by heating, stirring, change of pH, etc. and that this degradation tendency is increased as the molecular weight becomes higher. Namely, the conventinal technique accompanies side reactions such as degradation of hyaluronic acid to low molecule. Moreover, the haluronic acid derivative with high DS (10% or more) tends to cause stimulation in a living body, possibly damaging the outstanding intrinsic properties of a hyaluronic acid.

The above-described patent application further presents an example in which cinnamic acid was introduced into the carboxyl group of a glycosaminoglycan using a diamine as the spacer but this method is not satisfactory in the selectivity of the cinnamic acid substitution reaction.

Makromol. Chem. (1990), 191(12), 2985-91, describes photoreactive cellulose derivatives with comb-like structures which represent modifications of O-trialkylsilylcellulose with cinnamic acid-containing side chains.

### SUMMARY OF THE INVENTION

A first object of this invention is to provide a cinnamic acid derivative by introduction of a spacer into photodimerizable cinnamic acid.

A second object of the invention is to provide a cinnamic acid-polymer derivative, which is photodimerizable with high sensitivity and high efficiency, through the introduction of said cinnamic acid derivative into a hyaluronic acid host polymer and a crosslinked cinnamic acid polymer-derivative available on photocrosslinking said cinnamic acid-polymer derivative.

As the result of intensive research, the inventors of this invention succeeded in accomplishing the abovementioned objects by way of the following invention;

A cinnamic acid derivative represented by any of the formula (1) or (3) or a salt thereof:

$$R^1\text{-A-H} \quad (1)$$

$$R^2\text{-C-H} \quad (3)$$

wherein $R^1$ represents a group of the formula (4):

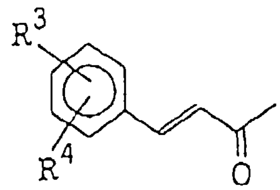

wherein $R^3$ and $R^4$ independently represent a hydrogen atom, a nitro group, an amino group, a hydroxy group or a $C_{1-4}$ alkoxy group;
$R^2$ represents a group of the formula (5):

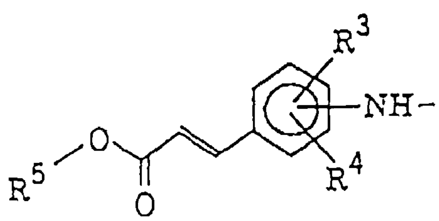

wherein $R^3$ and $R^4$ are as defined above; $R^5$ represents a $C_{1-4}$ alkyl group;
A-H represents an intramolecularly amino- and hydroxy-containing compound residue represented by any of the formulas (6)-(9):

$$\text{-O-(CH}_2\text{)n-NH}_2 \quad (6)$$

wherein n represents a whole number of 3-18,

$$\text{-(O-CH}_2\text{CH}_2\text{)m-NH}_2 \quad (7)$$

wherein m represents a whole number of 2-10,

$$\text{-O-CHR}^6\text{CH(COOR}^7\text{)-NH}_2 \quad (8)$$

wherein $R^6$ represents a hydrogen atom or a $C_{1-4}$ alkyl group; $R^7$ represents a $C_{1-4}$ alkyl group,

$$\text{-O-(CH}_2\text{)}\ell\text{-NHCO-CHR}^8\text{-NH}_2 \quad (9)$$

wherein $\ell$ represents a whole number of 2-18; $R^8$ represents the side chain of an $\alpha$-amino acid residue;
C-H represents an amino acid residue of the formula (11) or (12):

$$-CO-(CH_2)k-NH_2 \quad (11)$$

wherein k is representing a whole number of 1-18,

$$-(COCHR^8NH)i-H \quad (12)$$

wherein i represents a whole number of 1-6; $R^8$ is as defined above under the proviso that $R^1$-A-H does not represent 0-cinnamoyl-DL-serine methyl ester hydrobromide and $R^2$-C-H does not represent 4-benzyloxy-L-phenylalanine 4-(2-methoxycarbonylvinyl)anilide hydrochloride or 3-phenoxy-DL-phenylalanine 4-(2-methoxycarbonylvinyl)anilide hydrochloride.

A cinnamic acid-polymer derivative of any of the formulas (13) or (15):

$$R^1-A-P^1 \quad (13)$$

$$R^2-C-P^1 \quad (15)$$

wherein $R^1$ and $R^2$ have the same meanings as defined in (1) or (3) A- represents an amino- and hydroxy-containing compound residue represented by any of the formulas (6')-(9'):

$$-O-(CH_2)n-NH \quad (6')$$

wherein n represents a whole number of 3-18,

$$-(O-CH_2CH_2)m-NH \quad (7')$$

wherein m represents a whole number of 2-10,

$$-O-CHR^6CH(COOR^7)-NH \quad (8')$$

wherein $R^6$ represents a hydrogen atom or a $C_{1-4}$ alkyl group;
$R^7$ represents a $C_{1-4}$ alkyl group,

$$-O-(CH_2)\ell-NHCO-CHR^8-NH \quad (9')$$

wherein $\ell$ represents a whole number of 2-18; $R^8$ represents the side chain of an $\alpha$-amino acid residue;
C- represents an amino acid residue of the formula (11') or (12'):

$$-CO-(CH_2)k-NH \quad (11')$$

wherein k is representing a whole number of 1-18,

$$-(COCHR^8NH)i \quad (12')$$

wherein i represents a whole number of 1-6; $R^8$ is as defined above;
$P^1$ represents a hyaluronic acid;
the A-$P^1$ linkage is the amide bond formed between the terminal amino group of the formula (6'), (7'), (8') or (9') and

the carboxyl group of $P^1$;
and the C-$P^1$ linkage is the amide bond formed between the terminal amino group of the formula (11') or (12') and the carboxyl group of $P^1$ wherein the degree of substitution of the cinnamic acid derivative is 0.05-5.0% per disaccharide unit of the hyaluronic acid having a number average molecular weight of 100,000-5,000,000.

A crosslinked cinnamic acid-polymer derivative produced by photodimerization of $R^1$ and $R^1$, $R^2$ and $R^2$, or $R^1$ and $R^2$ of the above-described cinnamic acid-polymer derivative to form a crosslinked cyclobutane ring.

A crosslinked cinnamic acid-hyaluronic acid derivative wherein the cinnamic acid derivative is one or more members selected from the group consisting of the above formula (1) or (3) or a salt thereof in which the formula (1) or (3) is bonded to the carboxyl group of hyaluronic acid via the amide bond and $R^1$ and $R^1$, $R^2$ and $R^2$, or $R^1$ and $R^2$ of the cinnamic acid-polymer derivative is photodimerized to form a crosslinked cyclobutane ring wherein the degree of substitution of the cinnamic acid derivative is 0.05-5.0% per disaccharide unit of the hyaluronic acid having a number average molecular weight of 100,000-5,000,000.

A process for producing the cinnamic acid-hyaluronic acid derivative which comprises dissolving a hyaluronic acid in water or in water containing a water-miscible organic solvent and reacting the carboxyl group of the hyaluronic acid with the amino group of the cinnamic acid derivative represented by the above formula (1) or (3), wherein the degree of substitution of the cinnamic acid derivative is 0.05-5.0% per disaccharide unit of the hyaluronic acid having a number average molecular weight of 100,000-5,000,000, in the presence of a water-soluble carbodiimide and an auxiliary condensing agent; and

A process for producing the crosslinked cinnamic acid-hyaluronic acid derivative which comprises dissolving the cinnamic acid-hyaluronic acid derivative obtained by the above process in water or a water-miscible organic solvent, removing the water or water-miscible organic solvent from the resulting solution, forming the photodimerizable hyaluronic acid derivative into a shaped article and irradiating the shaped article with ultraviolet light.

Since the polymer compound corresponding to above-described $P^1$ is a hyaluronic acid, this invention provides a photocrosslinkable hyaluronic acid derivative introduced with a cinnamic acid derivative corresponding to above-described formula (1) or (3) as a photodimerizable-crosslinkable compound at mean 0.0005-0.05 per constituent disaccharide unit of hyaluronic acid. Here, DS of the photocrosslinkable hyaluronic acid derivative having photodimerizable-crosslinkable group introduced at mean 0.0005-0.05 per constituent disaccharide unit of hyaluronic acid is 0.05-5.0%.

In this invention, the inventors found the reaction conditions without degradation of high molecular weight hyaluronic acid (100,000-5,000,000) to low molecule and enabling the introduction of a photodimerizable-crosslinkable group. Namely, it was found that even the hyaluronic acid derivative introduced with photodimerizable-crosslinkable group at 1-100 per mean 4,000 monosaccharide per molecule of hyaluronic acid can be crosslinked and insolubilized by irradiation of ultraviolet light for several minutes.

This invention provides technology that can afford a water-insoluble crosslinked hyaluronic acid derivative setting DS as very low as mentioned above by short tine irradiation of light compared with conventional technology.

<u>BRIEF DESCRIPTION OF THE DRAWINGS</u>

Fig. 1 shows concept illustrating the mutual photocrosslinking reaction of a cinnamic acid derivative;
Fig. 2 graphically shows the result obtained in Example 51, indicating the reactivity dependent on the spacer carbon number;
Fig. 3 graphically shows the result obtained in Example 52, indicating the relationship between water absorption rate and DS;
Fig. 4 graphically shows the result obtained in Example 53, indicating the relationship between tensile strength and DS;
Fig. 5 graphically shows the result obtained in Example 53, indicating the relationship between tensile strength and water absorption rate;
Fig. 6 is graphically shows the result obtained in Example 54, indicating the relationship between the reciprocal of tensile strength and water absorption rate;
Fig. 7 graphically shows the result obtained in Example 55, indicating the critical point of insolubilization;
Fig. 8 graphically shows the result obtaind in Example 56, indicating the relative reactivity of the moiety of the spacer other than the methylene chain; and
Fig. 9 graphically shows the result obtained in Example 57, indicating the relationships between DS and water absorption rate of various spacer structures other than the methylene chain.

DETAILED DESCRIPTION OF THE INVENTION

It is known that photodimerizable cinnamic acid and aminocinnamic acid compounds absorb ultraviolet (UV) light in a defined band around 280 nm to dimerize into truxillic acid or truxinic acid or derivatives thereof. This photodimerization reaction proceeds only under a certain quantity of ultraviolet radiation of the defined wavelength and does not proceed under ordinary light such as solar radiation. On this meaning, a photocrosslinked article obtainable by exposing a cinnamoylated glycosaminoglycan derivative, namely a compound available upon introduction of cinnamic acid into the functional group of a glycosaminoglycan, to ultraviolet irradiation is advantageous in that it can be provided by a clean photoreaction with high sensitivity and that the starting material, cinnamoylated glycosaminoglycan derivative, is easy to handle because it is hardly affected by sunlight and white light during storage.

However, in order to obtain a photocrosslinked article using the cinnamoylated glycosaminoglycan derivative previously developed as described in EP-A-0 554 898 or JP-A-Hei-6-73102, for example a hyaluronic acid with a number average molecular weight of 800,000, as the host polymer, it is necessary to introduce 0.1-4 units (DS 10-400%) of cinnamic acid per disaccharide unit and irradiate the resulting compound with ultraviolet light for at least 30 minutes.

The inventors of this invention discovered that introduction of a flexible spacer between a hyaluronic acid host polymer and cinnamic acid results in a marked enhancement of photocurability so that the photocrosslinking reaction proceeds satisfactorily even at a more reduced degree of substitution with photocroaslinking groups.

The photocrosslinkable hyaluronic acid derivative with a number average molecular weight of 100,000 - 5,000,000, which is a cinnamic acid-polymer derivative containing a cinnamic acid derivative having such a spacer structure, can undergo photocrosslinking reaction at a reduced degree of substitution of 0.0005-0.05 units per disaccharide unit (DS 0.05-5%) and even with a UV exposure time of as short as 1-8 minutes.

The cinnamic acid derivatives of formulas (1) and (3) are now described in detail.

In formulas (1) and (3) ($R^1$-A-H (1) and $R^2$-C-H (3)], A-H, and C-H are the residues having the above-mentioned spacer function.

In formula (1), $R^1$ represented by formula (4) denotes a cinnamic acid residue having a vinylene group capable of photodimerizing to form a cyclobutane ring and $R^3$ and $R^4$ independently represent hydrogen, nitro, amino, hydroxy or $C_{1-4}$ alkoxy, and hydrogen being preferred. The alkoxy that can be used includes methoxy, ethoxy, propoxy, isopropoxy, butoxy, isobutoxy and t-butoxy, etc. The positions of bonding of $R^3$ and $R^4$ to the benzene ring are not critical, except for any interaction of the substituent groups, steric hindrance and reduction of reactivity of the amino group.

A-H represents the residue of a compound having both an amino group and a hydroxyl group within the molecule as represented by any of formulas (6)-(9), which discharges the spacer function.

Thus, the formula $R^1$-A-H represents a cinnamic acid derivative in which the carbonyl group of cinnamic acid residue represented by $R^1$ is bonded to A-H and a spacer having an amino group is introduced into its terminus.

$$-O-(CH_2)_n-NH_2 \qquad (6)$$

wherein n represents a whole number of 3-18, preferably 4-8, more preferably, 5 or 6. If n is 2 or less, the cinnamic acid-polymer derivative (formula (13)) obtained by introduction of a compound of formula (1) into a host polymer shows only a low efficiency of photocrosslinking reaction. If n is 19 or greater, the efficiency of the reaction introducing a compound of formula (1) into the polymer is lowered.

The residue of the above formula (6) is derived from an aminoalcohol such as aminoethanol, aminopropanol, aminobutanol, aminopentanol, aminohexanol, aminooctanol, aminododecanol, etc.

When A-H is the residue of the formula (6) derived from an aminoalcohol, the cinnamic acid derivative $R^1$-A-H can also be represented by the formula (1'-1)

$$H-NH(CR^{'1}R^{'2})nOCOCH=CH-Ph \qquad (1'-1)$$

wherein R'1 and R'2 each represent a hydrogen atom, Ph represents a phenyl group which may have one or two substituents such as a $C_{1-4}$ alkoxy group, an amino group, a hydroxyl group or a nitro group, n has the same definition as in the formula (6).

Typical examples of these compounds include cinnamic acid 3-aminopropyl ester, cinnamic acid 4-aminobutyl ester, cinnamic acid 5-aminopentyl ester, cinnamic acid 6-aminohexyl ester, cinnamic acid 8-aminooctyl ester, cinnamic acid 12-aminododecyl ester, etc.

$$-(O\text{-}CH_2CH_2)_m\text{-}NH_2 \quad (7)$$

wherein m represents a whole number of 2-10, preferably 2-5, more preferably, 2 or 3. If m is 1 or less, the cinnamic acid-polymer derivative will show only a low efficiency of photocrosslinking reaction, while an m value of 11 or more, the efficiency of the reaction introducing a compound of formula (1) into a host polymer is lowered.

The residue of the above formula (7) is derived from a polyethylene glycolamine such as diethylene glycolamine, etc.

$$-O\text{-}CHR^6CH(COOR^7)\text{-}NH_2 \quad (8)$$

wherein $R^6$ represents hydrogen or lower alkyl, preferably H or methyl; $R^7$ represents lower alkyl.

The residue of the above formula (8) is derived from a lower alkyl ester of a hydroxyamino acid such as serine or threonine, for instance.

When A-H is the residue of the formula (8) derived from a hydroxyamino acid, typical examples of the cinnamic acid derivative $R^1$-A-H include O-cinnamoyl serine methyl ester, O-cinnamoyl threonine methyl ester, etc.

$$-O\text{-}(CH_2)_\ell\text{-}NHCO\text{-}CHR^8\text{-}NH_2 \quad (9)$$

wherein $\ell$ represents a whole number of 2-18, preferably 2-8, more preferably, 2 or 3; $R^8$ represents a side chain of an $\alpha$-amino acid residue and is preferably hydrogen, methyl or isobutyl.

In the above formula (9), the moiety $-CO\text{-}CHR^8\text{-}NH_2$ is specifically derived from glycine, alanine, leucine or the like and the moiety $-O\text{-}(CH_2)_\ell$ -NH- is specifically derived from aminoethanol, aminopropanol or the like.

In formula (3), $R^2$ represents an aminocinnamic acid ester residue containing a vinylene group capable of photodimerizing to form a cyclobutane ring and optionally having a substituent or substituents represented by $R^3$ and $R^4$ which are as defined hereinbefore and each is preferably hydrogen; $R^5$ represents a lower alkyl group and is preferably methyl. The positions of bonding of $R^3$ and $R^4$ to the benzene ring are not critical except for any interaction of the substituent groups, steric hindrance and reduction of reactivity of the amino group.

The lower alkyl group used herein means an alkyl group having 1 to 4 carbon atoms.

C-H represents an amino acid residue of formula (11) or (12), which discharges the spacer function.

$$-CO\text{-}(CH_2)_k\text{-}NH_2 \quad (11)$$

wherein k has the same meaning as defined hereinbefore.

The residue of the above formula (11) can be derived. from any of amino acids essentially similar to those mentioned for the residue of formula (10).

When C-H is the residue of the formula (11) derived from an amino acid, the cinnamic acid derivative $R^2$-C-H can also be represented by the formula (2'-1-1).

$$H\text{-}A'\text{-}NH\text{-}Ph\text{-}CH{=}CHCOCR'^3 \quad (2'\text{-}1\text{-}1)$$

wherein $R'^3$ is an alkyl group having 1 to 4 carbon atoms, A' represents $-NH(CR'^4R'^5)h'CO-$, wherein $R'^4$ and $R'^5$ represent a hydrogen atom, Ph represents a para-phenyl group which may have one or two substituents such as a $C_{1-4}$ alkoxy group, an amino group, a hydroxyl group or a nitro group and h' is a whole number of 1 to 12.

Typical examples of these compounds include 4-(4-aminobutanamido)cinnamic acid methyl ester, 4-(6-aminohexamido)cinnamic acid methyl ester, 4-(12-aminododecanamido)cinnamic acid methyl ester, etc.

In the formula (2'-1-1), $R'^4$ and $R'^5$ represent a hydrogen atom.

$$-(COCHR^8NH)_i\text{-}H \quad (12)$$

wherein i represents a whole number of 1-6, preferably 2-4, more preferably, 2 or 3; $R^8$ has the same meaning as defined hereinbefore and is preferably hydrogen.

The residue of the above formula (12) can be specifically derived from ordinary α-amino acids or oligomers thereof (e.g. glycine, glycylglycine, triglycine, etc.).

When C-H is the residue of the formula (12) derived from an amino acid, the cinnamic acid derivative $R^2$-C-H can also be represented by the formula (2'-1-2).

$$H\text{-}A''\text{-}NH\text{-}Ph\text{-}CH{=}CHCOOR'^{3} \qquad (2'\text{-}1\text{-}2)$$

wherein A" represents -(NHCR'$^4$R'$^5$CO)m'-; R'$^3$ and Ph has the same definition as in the formula (2'-1-1); and m' is a whole number of 1 to 6, preferably 1 to 3.

Typical examples of these compounds include glycyl-aminocinnamic acid methyl ester, glycylglycylaminocinnamic acid methyl ester, triglycylaminocinnamic acid methyl ester, etc.

In the formula (2'-1-2), R'$^4$ and R'$^5$ may independently represent a hydrogen atom or one may be a lower alkyl group, preferably having 1 to 4 carbon atoms.

[1] Synthesis of cinnamic acid derivatives

Processes for producing the cinnamic acid derivatives of formulas (1 ) and (3) are then described.

[1-1] The cinnamic acid derivative of formula (1) can be prepared, for example by the following reaction process.

In the following description, the moiety represented by -A-H in formula (1) is sometimes designated as -O-$A^1$-$NH_2$ for easier explanation of the reaction involved.

The objective compound of formula (1) can be obtained by subjecting the carboxyl group of a cinnamic acid derivative of the formula $R^1$-OH to react selectively with the hydroxyl group of a compound having both an amino group and a hydroxyl group ($H_2N$-$A^1$-OH). Generally, in order to react only said hydroxyl group efficiently and selectively, it is preferable to use the latter compound which has the amino group suitably protected beforehand (RaNH-$A^1$-OH). Then, the unprotected hydroxyl group of the said compound is subjected to form an ester linkage to provide an N-protected compound (1) Ra-NH-$A^1$-O-$R^1$. Removal of the amino-protecting group under suitable conditions gives the objective compound. Thus, by these two steps of esterification and deprotection, the objective cinnamic acid ester having a free amino group or a salt thereof can be obtained with good efficiency.

The synthetic reaction scheme involved is described below.

$R^1$-X+RaNH-$A^1$-OH

↓ esterification

RaNH-$A^1$-O-$R^1$

↓ deprotection

$H_2N$-$A^1$-$OR^1$ [formula (1): $R^1$-A-H]

or a salt thereof.

Each of these two steps is described in detail below.

<u>First Step</u>

The carboxyl group of a cinnamic acid derivative represented by the formula $R^1$-OH is first activated [for example, in the form of a cinnamic acid halide of formula $R^1$-X (e.g. cinnamoyl chloride), cinnamic anhydride $(R^1)_2O$, or a mixed acid anhydride $R^1$-O-R')] and this activated carboxyl group is subjected to react with the unprotected hydroxyl group of a compound having both an amino group and a hydroxyl group ($H_2N$-$A^1$-OH) (RaNH-$A^1$-OH) of which the amino group has been protected beforehand to form an ester linkage, thus giving a compound (1) having a protected amino group, viz. RaNH-$A^1$-O-$R^1$. This reaction is preferably conducted in the presence of an acylation catalyst such as an N,N-dialkylaminopyridine (e.g. 4-dimethylaminopyridine, 4-pyrrolidinopyridine, etc.) or the like, and a neutralizer for the byproduct acid (e.g. a tertiary amine such as pyridine, triethylamine, etc. or an inorganic base such as sodium

hydrogen carbonate).

The amino-protecting group that can be used in the above step is not particularly limited, provided that it can be removed under conditions not cleaving the cinnamic acid ester in the deprotection procedure in the second step.

Second Step

The amino-protecting group of the compound ($RaNH-A^1-O-R^1$) obtained in the above first step is removed under conditions not cleaving the cinnamic acid ester. The amino-protecting group that can be used includes t-butoxycarbonyl, benzyloxycarbonyl, and 9-fluorenylmethoxycarbonyl, etc. When a protective group such as t-butoxycarbonyl which can be removed by means of an acid is employed, the treatment with hydrogen chloride, hydrogen bromide, trifluoroacetic acid or the like simultaneously removes the amino-protecting group and produce the corresponding salt.

The hydrochloride of $R^1$-A-H, for example, can be synthesized in the following manner.

To the compound ($Boc-NH-A^1-OH$) employing t-butoxycarbonyl (Boc-) group as an N-protecting group which can be removed with an acid is added an organic solvent such as chloroform, etc. and, then under ice-cooling, an organic base such as triethylamine, etc., an acid halide of $R^1$-OH ($R^1$-X), and a basic catalyst (acylation catalyst) such as 4-dimethylaminopyridine, etc. are added in the order mentioned. After stirring at room temperature, the mixture is diluted with an organic solvent such as ethyl acetate and washed, in order, several times with each of a weakly acidic aqueous solution, water, a weakly alkaline aqueous solution, and with water, and a saturated aqueous sodium chloride solution in a separatory funnel. The organic layer is then dried over anhydrous sodium sulfate, etc. The resulting anhydrous sodium sulfate is then filtered off and the filtrate is concentrated under reduced pressure to provide the compound ($RaNH-A^1-O-R^1$) (First Step). To this compound ($RaNH-A^1-O-R^1$) is added an organic solvent solution of an acid, such as 1-5M HCl/dioxane, under ice-cooling and stirring to remove the amino-protecting group and, at the same time, to convert the product amine to the hydrochloride. An organic solvent for crystallization, such as ether, is then added and the resulting crystals are recovered by filtration. If required, this crystal crop is washed with an organic solvent and dried in vacuo to provide the compound ($R^1$-A-H hydrochloride) (Second Step).

[1-3] The cinnamic acid derivative of formula (3) can be produced by, for example, the following process.

In the following description, the moiety -C-H in formula (3) is sometimes designated as $-CO-C^1-NH_2$ for ease of explanation.

The objective compound $R^2$-C-H can be obtained by subjecting the amino group of an ester of aminocinnamic acid of the formula $R^2$-H to react selectively with the carboxyl group of the amino acid ($H_2N-C^1-COOH$) to form an amide linkage. In order to achieve this selective reaction with the carboxyl group of the amino acid, it is generally preferable to use an amino acid whose amino group has been previously protected, i.e. an N-protected amino acid ($RaNH-C^1-COOH$). Thus, the amino group of $R^2$-H reacts with the carboxyl group of $RaNH-C^1-COOH$ to form an amide linkage to give $RaNH-C^1-CO-R^2$; N-protected $R^2$-C-H (First Step). This compound is then deprotected under suitable conditions to provide the objective compound (Second Step). Thus, the objective compound can be prepared by the above two steps of amidation and deprotection. This synthetic reaction procedure is described as follows.

$RaNH-C^1-COOH + R^2-H$

↓ amidation

$RaNH-C^1-CO-R^2$

↓ deprotection

$R^2-C-H$

Each of these steps is described in detail below.

[First Step]

The carboxyl group of the N-protected amino acid ($RaNH-C^1-COOH$) is first activated (for example, in the form of N-protected amino acid halide represented by $RaNH-C^1-CO-X$; N-protected amino acid anhydride represented by $(RaNH-C^1-CO)_2O$; or mixed acid anhydride represented by $RaNH-C^1-CO-O-R'$) and then reacted with the amino group of the ester of aminocinnamic acid $R^2$-H in the presence of an organic base. The amino-protecting group of said

N-protected amino acid is not particularly restricted, provided that the ester linkage of said ester of aminocinnamic acid is not cleaved under the conditions of deprotection of the protecting group. Preferred examples of the amino-protecting group are t-butoxycarbonyl, benzyloxycarbonyl, 9-fluorenylmethoxycarbonyl, etc. Particularly preferred is t-butoxycarbonyl. This reaction mixture is washed with an acid and an alkali to provide compound $RaNH-C^1-CO-R^2$ in a separatory funnel. Depending on the case, this product is preferably further purified by a routine purification procedure such as recrystallization and column chromatography.

[Second Step]

The amino-protecting group of the compound ($RaNH-C^1-CO-R^2$) obtained in the First Step is deprotected under suitable conditions to provide the objective compound $R^2$-C-H. Since the ester residue of the ester of aminocinnamic acid is a lower alkyl group, it is not appropriate to carry out deprotection by alkali saponification. Preferred is deprotection with an acid or by hydrogenolysis. More preferred is deprotection with an acid which forms a suitable salt at the same time. For example, in case of the use of t-butoxycarbonyl for the protective group, deprotection and salt formation using hydrogen chloride, hydrogen bromide, trifluoroacetic acid or the like acid can be utilized. In consideration of the introduction of the compound into a host polymer in the next step, salt formation with an acid having a carboxyl group, such as trifluoroacetic acid, is not desirable. Thus, deprotection and salt formation using a hydrogen halide, particularly hydrogen chloride, is preferred.

A specific example of procedure for the synthesis of compound ($R^2$-C-H) is described below.

The compound ($RaNH-C^1-COOH$) is dissolved in an organic solvent such as chloroform, and then under ice-cooling an organic base such as triethylamine and a condensing agent such as dimethylphosphinothioyl chloride or pivaloyl chloride are added successively. The mixture is stirred at room temperature. To this solution, the compound $R^2$-H or a salt thereof and a base such as triethylamine are added under ice-cooling and the mixture is stirred at room temperature for tens of minutes to tens of hours. After completion of the reaction, the organic solvent is distilled off under reduced pressure and the residue is diluted with an organic solvent such as ethyl acetate and, using a separatory funnel, washed successively several times with a weakly acidic aqueous solution, water, a weakly alkaline aqueous solution, and with water, and saturated aqueous sodium chloride solution. The organic layer is then dried over anhydrous sodium sulfate or the like. The sodium sulfate is filtered off and the filtrate is concentrated under reduced pressure to give the compound ($RaNH-C^1-CO-R^2$). To this compound ($RaNH-C^1-CO-R^2$) is added a solution of an acid in an organic solvent, e.g. 1-5M HCl/dioxane, under ice-cooling and the mixture is stirred. After completion of the reaction, an organic solvent such as ether is added and the resulting crystals are collected by filtration, washed with an organic solvent, and dried in vacuo to provide the hydrochloride of the compound ($R^2$-C-H).

[2] Synthesis of cinnamic acid-polymer derivatives

The above spacer-incorporated cinnamic acid derivatives of formulas (1) and (3) are all new compounds. By reacting any of these derivatives with a polymer compound (host polymer) having a functional group capable of reacting with its functional group, a cinnamic acid-polymer derivative of the corresponding formulas(13) and (15) can be obtained.

$$R^1\text{-A-}P^1 \qquad (13)$$

$$R^2\text{-C-}P^1 \qquad (15)$$

wherein $R^1$, $R^2$, A, and C are as defined hereinbefore; $P^1$ represents a carboxy-containing polymer residue;
the A-$P^1$ linkage is the amide bond formed between the terminal amino group of the residue of formula (6) - (9) and the carboxyl group of $P^1$;
and the C-$P^1$ linkage is the amide bond formed between the terminal amino group of the residue of formula (11) or (12) and the carboxyl group of $P^1$.

The host polymer compound containing $P^1$ is a hyaluronic acid.

Purification following the reaction can be achieved by a routine procedure such as ethanol precipitation, dialysis or the like. After drying, DS can be determined by observing $^1$H-NMR (nuclear magnetic resonance) integral intensity or absorbance at 280 nm.

[2-1] The cinnamic acid-polymer derivative of formula (13) or (15) can be produced by dissolving the polymer containing the residue $P^1$ in either water or water containing a water-miscible organic solvent and reacting it with a cinnamic acid

derivative of formula (1) or (3) in the presence of a water-soluble carbodiimide and an auxiliary condensing agent. The specific example of procedure for introduction of the cinnamic acid derivative is as follows. Said polymer is dissolved in either water or water containing a water-miscible organic solvent and, the solution is gently stirred at a constant temperature of about 0°C-40°C (usually, 0°C-35°C), said water-soluble carbodiimide, said auxiliary condensing agent, and said cinnamic acid derivative are successively added.

The water-miscible organic solvent mentioned above includes dioxane, dimethylformamide (DMF), N-methylpyrrolidone, acetamide, alcohol (e.g. methanol, ethanol, etc.), pyridine, etc.

The proportion of the water-miscible organic solvent in said water containing a water-miscible organic solvent (organic solvemt mixing ratio = ΔO) can be expressed as follows.

$$\Delta O\ (\%) = 100 \times \text{volume of water-miscible organic solvent/volume of water containing the water-miscible organic solvent}$$

The value of ΔO is approximately 0-75%, preferably 30-50%.

The auxiliary condensing agent that can be used includes N-hydroxysuccinimide (HOSu), N-hydroxybenzotriazole (HOBt), etc. The auxiliary condensing agent not only functions to activate the carboxyl group of the polymer but also functions to preclude the undesired O-N-acyl rearrangement.

The water-soluble carbodiimide (WSC) that can be used includes 1-ethyl-3-(3-dimethylaminopropyl)carbodiimide hydrochloride (EDC), 1-ethyl-3-(3-dimethylaminopropyl)carbodiimide methiodide, 1-cyclohexyl-3-(2-morpholinoethyl)carbodiimide hydrochloride, etc.

[3] Preparation of crosslinked cinnamic acid-polymer derivatives

[3-1] The cinnamic acid-polymer derivative of this invention can be prepared in a variety of forms for submission to photocrosslinking reaction. For example, such forms are solutions, films, gels, finely divided particles, and so on. Since the hyaluronic acid host polymer compound to be used is a highly hydrophilic and good biocompatible substance, the cured articles prepared by subjecting said cinnamic acid-polymer derivative to a photocrosslinking reaction are suitable for a variety of medical uses (biological tissue adhesion prevention material, vascular prosthesis material, coating agent, base material for control-release pharmaceutical preparations, contact lens material, bandage material, etc.) and the photocrosslinking by ultraviolet irradiation can be performed after the cinnamic acid polymer-derivative has been processed into such end-use forms as described above depending on the application object.

For the processing of the cinnamic acid-polymer derivative of this invention into such various forms, any of known technologies can be employed. Such processing can be made by judicious selection and appropriate adjustment of the amount of water or the proportion of a water-miscible organic solvent (organic solvent mixing ratio) in a solution of the cinnamic acid-polymer derivative or a bulk solid of the cinnamic acid-polymer derivative, temperature and pressure according to the intended purpose.

In the preparation of such a crosslinked cinnamic acid polymer derivative suited for medical application, the objective photocrosslinked cinnamic acid-polymer article can be assured to be a sterile and substantially endotoxin-free status by taking care of the selection and handling to ensure a sterile state of the reagents, water and vessels for synthesis of starting materials and the water, vessels, and equipment for use in the molding and photocrosslinking of the cinnamic acid-polymer derivative.

Concretely, the reaction mixture in which the cinnamic acid-polymer derivative has been formed may be utilized as it is. As an alternative, the derivative formed is separated from the reaction mixture, purified and redissolved in, for example, water to provide an aqueous solution. A film can be manufactured by casting a solution (e.g. aqueous solution) of the cinnamic acid-polymer derivative and removing the solvent from said solution. Gels can be manufactured by, for example, immersing the above film in water. Finely divided particles can be obtained by physical grinding of said film or gel. [3-2] The crosslinked cinnamic acid-polymer derivative of this invention is such that the photodimerizable crosslinking groups of cinnamic acid-polymer derivative molecules form crosslinked cyclobutane rings and can be obtained by subjecting said cinnamic acid-polymer derivative to ultraviolet irradiation.

The type of ultraviolet light is not particularly limited. The light source having wavelength of 200-600 nm, preferably 200-450 nm, is used, The light can be passed through a ultraviolet filter (e.g., Pyrexglass (trade name) filter, etc.) to cut the light having wavelength which is not necessary for photocrosslinking.

The means for ultraviolet irradiation include technology using a high-pressure mercury vapor lamp or a metal halide lamp as the light source and generally said film, gel or finely divided particles of cinnamic acid-polymer derivative

is exposed to UV light at 250-450 nm for 1 to 10 minutes. Compared with the photocrosslinking of the conventional photocrosslinkable hyaluronic acid derivative having a high DS value which requires an irradiation time of 30 minutes, the technology of this invention features a marked reduction in the irradiation time. This effect may be attributed to the use of the spacer described hereinbefore (the residue A, or C in the cinnamic acid derivatives of formulas (1) and (3)).

Each of the cinnamic acid derivatives of formulas (1) and (3) exists generally in the trans form and when it is present at the intermolecular distance of approximately 4 Angstrom units (Å) and exposed to light of a specific wavelength of the UV region (280 nm), the derivative undergoes dimerization. However, at intermolecular distances longer than approximately 4 Å, the derivative does not dimerize and even if its molecule is excited, it isomerizes into a radiation-inert geometrical isomer, i.e. cis-form. While the cinnamic acid derivative of this invention forms a cinnamic acid-polymer derivative by introduction into a polymer compound, the photodimerization reaction on the chain of the introduced host polymer compound, is identical, in principle, to the above-mentioned dimerization reaction. Therefore, it is important that molecules of the cinnamic acid derivative should first exist at said dimerizable intermolecular distance in the photodimerization reaction.

In this invention, the molecules of one or more cinnamic acid-polymer derivatives selected from among those of formulas (13) and (15), namely molecules of mutual compound (13), mutual compound (15), compounds (13) and (15), or $R^1$ and $R^1$, $R^2$ and $R^2$, or $R^1$ and $R^2$ of compounds (13) and (15), are photodimerized to form a crosslinked cyclobutane ring and thereby provide a crosslinked cinnamic acid-polymer derivative. Where photocrosslinking is to occur between compounds of the same formula for compounds of formulas (13) and (15), the concrete compounds to be submitted to the reaction may be the same compound or different compounds.

The crosslinked cyclobutane ring of the crosslinked cinnamic acid-polymer derivative of this invention may generally exist as two structural isomers, namely the truxinic acid derivative and the truxillic acid derivative, and each of them may exist in a plurality of stereoisomers. The crosslinked cinnamic acid derivative of the invention may be in any of such structures.

For example, while the photocrosslinking of the cinnamic acid-polymer derivative can be effected by UV irradiation after the derivative has been made into a film by the casting method, the reactivity for this photocrosslinking reaction appears to be governed by the molecular orientation obtained in the casting process.

A schematic illustration of concept of the photoreaction for the formation of the crosslinked cinnamic acid-polymer derivative of this invention is presented in Fig. 1. Compared with the case in which cinnamic acid has been introduced directly into a host polymer chain, the reactivity for photodimerization is enhanced by the freedom of the interposed spacer. This is not a sole factor, however, and the hydrophobicity of the spacer also has a great influence on the photoreactivity.

Such a spacer includes amino acids or derivatives thereof, peptides, aminoalcohols.

While Fig. 1 is a schematic presentation of the photocrosslinking reaction according to this invention, in the figure reference numeral 1 indicates the cinnamic acid-polymer derivative. This cinnamic acid-polymer derivative 1 is a compound combined a polymer compound 2 with cinnamic acid derivative, and this cinnamic acid derivative comprises a trans-cinnamic acid 4 molecule bound to a spacer 3. When this cinnamic acid-polymer derivative 1 is exposed to ultraviolet radiation (UV), the photocrosslinking groups of adjacent trans-cinnamic acid molecules are bound to each other to form a cyclobutane ring and gives a dimer 6. Thus, the cinnamic acid-polymer derivative 1 is converted to the crosslinked cinnamic acid-polymer derivative 5. Some cinnamic acid molecules remain unreacted as cis~cinnamic acid 7 which is inert to the photocrosslinking reaction.

When a cinnamic acid derivative is introduced into a hydrophilic hyaluronic acid polymer and the resulting compound is molded (e.g. cast into a film) with the elimination of water, a high hydrophobicity of the cinnamic acid derivative results in mutual attraction and aggregation of the molecules through hydrophobic binding. Thus, the derivative tends to take a molecular orientation which favors photodimerization. Therefore, an increase in the hydrophobicity of a cinnamic acid derivative can be a factor in enhancement of the reactivity for photodimerization. The photodimerization reaction between the crosslinking groups on the cinnamic acid-polymer derivative results in the construction of a three-dimensional network structure due to the crosslinked cyclobutane rings of the cinnamic acid-polymer derivative. The crosslink density of such a network structure is governed by the photodimerizability of the cinnamic acid derivative. A difference in crosslink density influences on the water absorption rate and strength of the crosslinked product. Generally the higher is the crosslink density, the lower is the water absorption rate and the higher is the strength obtained. Therefore, even if such crosslinking groups are introduced into a host polymer at the same degree of substitution and the resulting compound is irradiated with ultraviolet light for the same time period, differences in various physical properties are ensured according to difference of spacers. Therefore, the physical characteristics of the crosslinked cinnamic acid-polymer derivative can be controlled by a judicious selection of the proper spacer as well.

The use of hyaluronic acid, which is a biogenic substance, as the host polymer for introduction of a cinnamic acid derivative is advantageous for medical applications in the sense that the inherent characteristics of the hyaluronic acid can be exploited and the risk of damage to the bio-body can then be virtually avoided.

Moreover, that a highly sensitive cinnamic acid derivative could be obtained by interposing a spacer has

enabled the production of a crosslinked cinnamic acid-polymer derivative at a low degree of substitution of crosslinking group and in a reduced UV exposure time. This implies that the physical characteristics of a crosslinked cinnamic acid-polymer derivative can be controlled at a degree of substitution which is so low as to be substantially negligible in terms of the effect on bio-body. Therefore, the intrinsic properties of the hyaluronic acid host polymer can be almost fully retained.

The following examples are intended to describe this invention in further detail and should by no means be construed as limiting the scope of the invention. Unless otherwise indicated herein, all parts, percents, ratios and the like are by weight.

[Synthesis of Cinnamic Acid Derivatives]

In the following description, Boc means t-butoxycarbonyl.

[1-1] Examples 1-6

Synthesis of a compound corresponding to formulas (1) and (6) as represented by the formula:

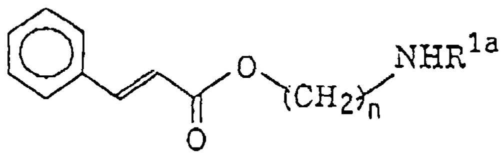

(wherein $R^1$ represents a group of the formula (4), $R^3$=$R^4$=H, and $R^{1a}$=H or an amino-protecting group)

EXAMPLE 1

Synthesis of 3-Aminopropyl Cinnamate [compound (1a-1), n=3, $R^{1a}$=H] Hydrochloride

1-1: Synthesis of [compound (1-1), n=3, $R^{1a}$=Boc]

To 1.21 g (6.9 mmol) of t-butoxycarbonyl-3-aminopropanol was added 6 ml of chloroform and, then under ice-cooling, 956 μl (6.9 mmol) of triethylamine, 1.15 g (6.9 mmol) of cinnamoyl chloride, and 253 mg (2.1 mmol) of 4-dimethylaminopyridine were added successively. The mixture was stirred at room temperature for 20 minutes. This reaction mixture was diluted with ethyl acetate and washed successively twice with 5% aqueous solution of citric acid, water, 5% aqueous solution of sodium hydrogen carbonate, and with water, and a saturated aqueous solution of sodium chloride in a separatory funnel. The organic layer was dried over anhydrous sodium sulfate. The sodium sulfate was then filtered off and the filtrate was concentrated under reduced pressure. The residual white solid was washed with hexane and dried in vacuo to provide 1.38 g (yield 65%) of compound (1-1).

1-2: Synthesis of 3-aminopropyl cinnamate [compound (1a-1), n=3, $R^{1a}$=H] hydrochloride

To 860 mg (2.8 mmol) of compound (1-1) was added 6 ml of 4M HCl/dioxane solution under ice-cooling and the mixture was stirred at room temperature for 35 minutes. To this reaction mixture was added ether and the resulting crystals were filtered off, washed with ether, and dried in vacuo to provide compound (1a-1) as white crystals. Yield 76%, m. p. 115.2-116.3°C.

$^1$H-NMR(400MHz, $D_2O$) δ(ppm) = 2.16 (2H, quant, $H_2NCH_2\underline{CH_2}CH_2O$-), 3.21 (2H, t, $H_2N\underline{CH_2}CH_2CH_2O$-), 4.37 (2H, t, $H_2NCH_2CH_2\underline{CH_2}O$-), 6.62 (1H, d, -CH=$\underline{CH}$CO-), 7.52 (3H, m, Aromatic H, 3,4,5 positions), 7.72 (2H, dd, Aromatic H, 2,6 positions), 7.80 (1H, d, -$\underline{CH}$=CHCO-)

EXAMPLE 2

Synthesis of 4-Aminobutyl Cinnamate [compound (1a-2), n=4, $R^{1a}$=H] Hydrochloride

2-1: Synthesis of [compound (1-2), n=4, $R^{1a}$=Boc]

The title compound was synthesized following essentially the procedure described in Example 1-1. Yield 93%.

2-2: Synthesis of 4-aminobutyl cinnamate [compound (1a-2), n=4, $R^{1a}$=H] hydrochloride

The title compound was synthesized following essentially the procedure described in Example 1-2. Yield 88%, m. p. 91.2-92.4 °C.

$^1$H-NMR(400MHz, $D_2O$)δ(ppm) = 1.85 (4H, m, $H_2NCH_2\underline{(CH_2)_2}CH_2O$-), 3.10 (2H, t, $H_2N\underline{CH_2}(CH_2)_3O$-), 4.30 (2H, t, $H_2N(CH_2)_3\underline{CH_2}O$-), 6.83 (1H, d, -CH=$\underline{CH}$CO-), 7.53 (3H, m, Aromatic H, 3,4,5 positions), 7.70 (2H, dd, Aromatic H, 2,6 positions), 7.80 (1H, d, -$\underline{CH}$=CHCO-)

EXAMPLE 3

Synthesis of 5-Aminopentyl Cinnamate [compound (1a-3), n=5, $R^{1a}$=H] Hydrochloride

3-1: Synthesis of [compound (1-3), n=5, $R^{1a}$=Boc]

The title compound was synthesized following essentially the procedure described in Example 1-1. Yield 100%.

3-2: Synthesis of 5-aminopentyl cinnamate [compound (1a-3), n=5, $R^{1a}$=H] hydrochloride

The title compound was synthesized following essentially the procedure described in Example 1-2. Yield 88%, m. p. 150.3-153.4°C.

$^1$H-NMR(400MHz, $D_2O$) δ(ppm)=1.52 (2H, quant, $H_2NCH_2CH_2\underline{CH_2}CH_2O$-), 1.70-1.86 (4H, m, $H_2NCH_2\underline{CH_2}CH_2\underline{CH_2}CH_2O$-), 3.05 (2H, t, $H_2N\underline{CH_2}$ $(CH_2)_4O$-), 4.29 (2H, t, $H_2N(CH_2)_4\underline{CH_2}$-O-), 6.61 (1H, d, -CH=$\underline{CH}$-CO-), 7.51 (3H, m, Aromatic H, 3,4,5 positions), 7.69 (2H, d, Aromatic H, 2,6 positions), 7.78 (1H, d, -$\underline{CH}$=CHCO-)

EXAMPLE 4

Synthesis of 6-Aminohexyl Cinnamate [Compound (1a-4), n=6, $R^{1a}$=H] Hydrochloride

4-1: Synthesis of [compound (1-4), n=6, $R^{1a}$=Boc]

The title compound was synthesized following essentially the procedure described in Example 1-1. Yield 99%.

4-2: Synthesis of 6-aminohexyl cinnamate [compound (1a-4), n=6, $R^{1a}$=H] hydrochloride

The title compound was synthesized following essentially the procedure described in Example 1-2, Yield 86%, m. p. 98.8-100.4°C.

$^1$H-NMR (400MHz, $D_2O$) δ(ppm)=1.48-1.53 (4H, m, $H_2NCH_2CH_2\underline{(CH_2)_2}CH_2CH_2O$-), 1.63-1.83 (4H, m, $H_2NCH_2\underline{CH_2}(CH_2)_2\underline{CH_2}CH_2O$-), 3.02 (2H, t, $H_2N\underline{CH_2}(CH_2)_5O$-), 4.28 (2H, t, $H_2N(CH_2)_5\underline{CH_2}O$-), 6.60 (1H, d, -CH=$\underline{CH}$-CO-), 7.53 (3H, m, Aromatic H, 3,4,5 positions), 7.68 (2H, d, Aromatic H, 2,6 positions), 7.76 (1H, d, -$\underline{CH}$=CHCO-)

EXAMPLE 5

Synthesis of 8-Aminooctyl Cinnamate [compound (1a-5), n=8, $R^{1a}$=H] Hydrochloride

5-1: Synthesis of [compound (1-5), n=8, $R^{1a}$=Boc]

The title compound was synthesized following essentially the procedure described in Example 1-1. Yield 87%.
5-2: Synthesis of 8-aminooctyl cinnamate [compound (1a-5), n=8, $R^{1a}$=H] hydrochloride
The title compound was synthesized following essentially the procedure described in Example 1-2. Yield 88%,

m. p. 86.5-87.3°C.

$^1$H-NMR(400Mz, $D_2O$) δ(ppm)=1.31-1.48 (8H, m, $H_2NCH_2CH_2(\underline{CH_2})_4CH_2CH_2O$-), 1.62-1.79 (4H, m, $H_2NCH_2\underline{CH_2}(CH_2)_4\underline{CH_2}CH_2O$-), 2.99 (2H, t, $H_2N\underline{CH_2}(CH_2)_7O$-), 4.26 (2H, t, $H_2N(CH_2)_7\underline{CH_2}O$-), 6.58 (1H, d, -CH=$\underline{CH}$-CO-), 7.52 (3H, m, Aromatic H, 3,4,5 positions), 7.68 (2H, d, Aromatic H, 2,6 positions), 7.76 (1H, d, -$\underline{CH}$=CHCO-)

EXAMPLE 6

Synthesis of 12-Aminododecyl Cinnamate [compound (1a-6), n=12, $R^{1a}$=H] Hydrochloride

6-1: Synthesis of [compound (1-6), n=12, $R^{1a}$=Boc]

The title compound was synthesized following essentially the procedure described in Example 1-1. Yield ca 100%.

6-2: Synthesis of 12-aminododecyl cinnamate [compound (1a-6), n=12, $R^{1a}$=H] hydrochloride

The title compound was synthesized following essentially the procedure described in Example 1-2. Yield 82%, m. p. 90.7-93.1°C.

$^1$H-NMR(400MHz, $D_2O$) δ(ppm)=1.24-1.50 (16H, m, $H_2NCH_2CH_2(\underline{CH_2})_8CH_2CH_2O$-), 1.64 (2H, quant, $H_2NCH_2\underline{CH_2}(CH_2)_{10}O$-), 1.76 (2H, quant, $H_2N(CH_2)_{10}\underline{CH_2}CH_2O$-), 2.96 (2H, t, $H_2N\underline{CH_2}(CH_2)_{11}O$-), 4.28 (2H, t, $H_2N(CH_2)_{11}\underline{CH_2}O$-), 6.61 (1H, d, -CH=$\underline{CH}$CO-), 7.53 (3H, m, Aromatic H, 3,4,5 positions), 7.68 (2H, d, Aromatic H, 2,6 positions), 7.76 (1H, d, -$\underline{CH}$=CHCO-)

[1-2] Synthesis of a compound corresponding to formulas (1) and (7) as represented by the formula:

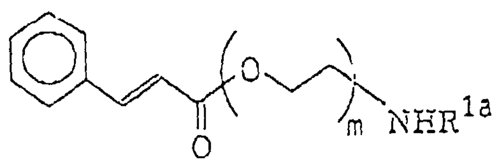

(wherein $R^1$ represents a group of the formula (4), $R^3$=$R^4$=H)

EXAMPLE 7

Synthesis of 2-(2-Aminoethoxy)ethyl Cinnamate [compound (1a-7), m=2, $R^{1a}$=H] Hydrochloride

7-1: Synthesis of [compound (1-7), m=2, $R^{1a}$=Boc]

Using Boc-$NH(CH_2CH_2O)_2H$ in lieu of Boc-$NH(CH_2)_3OH$, the title compound was synthesized following essentially the procedure described in Example 1-1. Yield 85%.

7-2: Synthesis of 2-(2-aminoethoxy)ethyl cinnamate [compound (1a-7), m=2, $R^{1a}$=H] hydrochloride

Using compound (1-7) in lieu of compound (1-1), the title compound was synthesized following essentially the procedure described in Example 1-2. Yield ca 100%, m. p. 80.8-82.5°C.

$^1$H-NMR(400MHz, $D_2O$) δ(ppm)-3.26 (2H, t, $H_2\underline{CH_2}CH_2O$-), 3.84 (2H, t, $H_2NCH_2\underline{CH_2}O$-), 3.88 (2H, t, -$O\underline{CH_2}CH_2OCO$-), 4.40 (2H, t, -$O\underline{CH_2}CH_2OCO$-), 6.54 (1H, d, -CH=$\underline{CH}$CO-), 7.49 (3H, m, Aromatic H, 3,4,5 positions), 7.64 (2H, m, Aromatic H, 2,6 positions), 7.73 (1H, d, -$\underline{CH}$=CHCO-)

[1-3] Synthesis of a compound corresponding to formulas (1) and (8) as represented by the formula

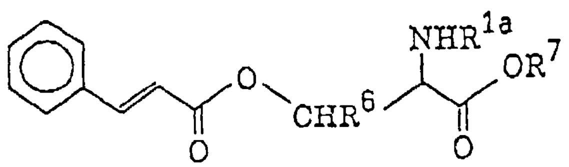

(wherein $R^1$ represents a group of the formula (4), $R^3=R^4=H$; and in the formula (8), $R^6=H$, $R^7=CH_3$)

EXAMPLE 8

Synthesis of Q-Cinnamoyl Serine Methyl Ester [compound (1a-8), $R^{1a}=H$] Hydrochloride

8-1: Synthesis of [compound (1-8), $R^{1a}$=Boc]

In 9 ml of chloroform was dissolved 1.93 g (8.8 mmol) of t-butoxycarbonylserine methyl ester and, then under ice-cooling, a solution of 2.94 g (10.6 mmol) of cinnamic acid anhydride in 10 ml chloroform, 1.46 ml (10.6 mmol) of triethylamine, and a solution of 645 mg (4.4 mmol) of 4-dimethylaminopyridine in 2 ml chloroform were added successively. The mixture was stirred at room temperature for 35 minutes and, then, concentrated under reduced pressure to reduce liquid volume. This solution was diluted with ethyl acetate and washed successively twice with 5% citric acid, once with water, twice with a 5% aqueous solution of sodium hydrogen carbonate, once with water, and twice with a saturated aqueous solution of sodium chloride. The organic layer was dried over anhydrous sodium sulfate for 1 hour. The sodium sulfate was then filtered off and the filtrate was concentrated under reduced pressure. To the residue was added ether and the resulting crystal crop was recrystallized from ethyl acetate-petroleum ether to provide 2.10 g (yield 68%) of white crystals of compound (1-8).

Structure was confirmed by $^1$H-NMR.

$^1$H-NMR(400MHz, $CDCl_3$) δ(ppm)=1.45 (9H, s, Boc-), 3.80 (3H, s, $-COOCH_3$), 4.51 (2H, dd, $\beta$-$CH_2$), 4.65 (1H, br, $\alpha$-CH), 5.38 (1H, br, CONH), 6.40 (1H, d, PhCH-<u>CH</u>-), 7.45 (5H, m, Ph-), 7.70 (1H, s, Ph<u>CH</u>=CH-)

8-2: Synthesis of O-cinnamoyl serine methyl ester [compound (1a-8), $R^{1a}=H$] hydrochloride

Under ice-cooling, trifluoroacetic acid was added to 1.40 g (4.2 mmol) of compound (1-8) up to the level where the crystals were fully immersed and the mixture was allowed to stand for 30 minutes. Then, 1.1 ml of 4M HCl/dioxane solution was added. After crystallization by addition of hexane, the crystal crop was filtered off and washed with ether-hexane on a glass filter to provide 1.39 g (yield 91%) of compound (1a-8) as white crystals. m.p. 144.5-147.0°C.

$^1$H-NMR(400MHz, $D_2O$) δ(ppm)=3.92 (3H, s, $-COOCH_3$), 4.58-4.75 (3H, dd, Ser $\alpha$-H, $\beta$-H), 6.80 (1H, d, -CH=<u>CH-</u>CO-), 7.50 (3H, m, Aromatic H, 3,4,5 positions), 7.68 (2H, d, Aromatic H, 2,6 positions), 7.82 (1H, d, -<u>CH</u>=CHCO-)

[1-4] Examples 9-11: Synthesis of a compound corresponding to formulas (1) and (9) as represented by the following formula

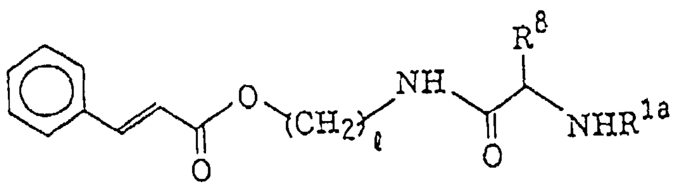

(wherein $R^1$ represents a group of the formula (4), $R^3=R^4=H$)

EXAMPLE 9

Synthesis of [compound (1a-9), ℓ=2, $R^{1a}$=H, $R^8$=H] Hydrochloride

In 2 ml of chloroform was dissolved 175 mg (1.0 mmol) of t-butoxycarbonylglycine and, then under ice-cooling, 139 μl (1.0 mmol) of triethylamine and 1.0 ml (1.0 mmol) of 1M dimethylphosphinothioyl chloride/chloroform solution were added. The mixture was stirred at room temperature for 20 minutes. Then, 139 μl (1.0 mmol) of triethylamine and a previously prepared solution of 2-aminoethyl cinnamate hydrochloride (1.0 mmol) and 139 μl (1.0 mmol) triethylamine in 2 ml of chloroform were added under ice-cooling and the mixture was stirred at room temperature for 2 hours. After completion of the reaction, 4 ml of methanol and 1 ml of aqueous ammonia were added and the mixture was stirred for 30 minutes. This solution was once concentrated under reduced pressure once and the residue was diluted with ethyl acetate and washed successively twice with water, a 5% aqueous sodium hydrogen carbonate solution, water, a 5% aqueous citric acid solution, and with water, and a saturated aqueous sodium chloride solution. The organic layer was dried over anhydrous sodium sulfate and concentrated under reduced pressure to provide [compound (1-9), ℓ=2, $R^{1a}$=Boc, R8=H]. To this compound was added 3 ml of 4M HCl/dioxane solution under ice-cooling, and the mixture was stirred at room temperature for 30 minutes and concentrated under reduced pressure to provide 106 mg (yield 35%) of the objective compound (1a-9) as white solid. m.p. 162.5-165.8°C.

$^1$H-NMR(400MHz, $D_2O$) δ(ppm)=3.83 (2H, s, -NH<u>$CH_2$</u>CO-), 3.66 (2H, t, -NH<u>$CH_2$</u>$CH_2$O-), 4.38 (2H, t, -NH$CH_2$<u>$CH_2$</u>O-), 6.62 (1H, d, -CH=<u>CH</u>CO-), 7.53 (3H, m, Aromatic H, 3,4,5 positions), 7.70 (2H, d, Aromatic H, 2,6 positions), 7.81 (1H, d, -<u>CH</u>=CHCO-)

EXAMPLE 10

Synthesis of [compound (1a-10), ℓ=2, $R^{1a}$=H, $R^8$=$CH_3$] hydrochloride

Using t-butoxycarbonylalanine in lieu of t-butoxycarbonylglycine, the title compound was synthesized following essentially the procedure described in Example 9. Yield 45%.

$^1$H-NMR(400MHz, $D_2O$) δ(ppm)=2.53 (3H, d, -$CH_3$), 3.55 (1H, dt, -NH<u>$CH_2$</u>$CH_2$O-), 3.72 (1H, dt, -NH<u>$CH_2$</u>$CH_2$O-), 4.08 (1H, qualt, Ala α-H), 4.36 (2H, t, -NH$CH_2$<u>$CH_2$</u>O-), 6.58 (1H, d, -CH=<u>CH</u>CO-), 7.50 (3H, m, Aromatic H, 3,4,5 positions), 7.67 (2H, d, Aromatic H, 2,6 positions), 7.76 (1H, d, -<u>CH</u>=CHCO-)

EXAMPLE 11

Synthesis of [compound (1a-11), ℓ=2, $R^{1a}$=H, $R^8$=$(CH_3)_2CHCH_2$-] Hydrochloride

Using t-butoxycarbonylleucine in lieu of t-butoxycarbonylglycine, the title compound was synthesized following essentially the procedure described in Example 9. Yield 58%.

$^1$H-NMR(400MHz, $D_2O$)δ(ppm)=0.90 (6H, dd, -CH<u>(CH3)2</u>), 1.55-1.80 (3H, m, -<u>$CH_2CH$</u>$(CH_3)_2$), 3.44 (1H, dt, -NH<u>$CH_2$</u>$CH_2$O-), 3.88 (1H, dt, -NH<u>$CH_2$</u>$CH_2$O-), 3.99 (1H, t, Leu α-H), 4.39 (2H, t, -NH$CH_2$<u>$CH_2$</u>O-), 6.60 (1H, d, - CH=<u>CH</u>-CO-), 7.51 (3H, m, Aromatic H, 3,4,5 positions), 7.70 (2H, d, Aromatic H, 2,6 positions), 7.78 (1H, d, - CH-CHCO-)

[3-1] Examples 12-14: Synthesis of a compound corresponding to formulas (3) and (11) as represented by the formula:

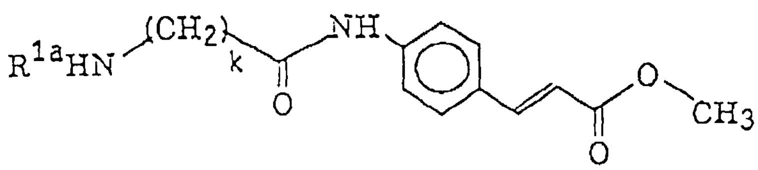

(wherein $R^2$ represents a group of the formula (3), and in the formula (5), $R^3$=$R^4$=H, $R^5$=$CH_3$)

EXAMPLE 12

Synthesis of Methyl 4-(4-Aminobutyrylamino)cinnamate Hydrochloride [compound (3a-1), k=3, $R^{1a}$=H]

12-1: Synthesis of [compound (3-1), k=3, $R^{1a}$=Boc]

In 3 ml of chloroform was dissolved 2.02 g (10 mmol) of t-butoxycarbonyl-γ-aminobutyric acid and, under ice-cooling, 1.38 ml (10 mmol) of triethylamine and 1.28 g (10 mmol) of dimethylphosphinothioyl chloride were successively added. The mixture was stirred at room temperature for 10 minutes. Under ice-cooling, 532 mg (3 mmol) of methyl p-aminocinnamate hydrochloride and 3 ml of a solution of 417 μl (3 mmol) of triethylamine in chloroform were added, followed by addition of a further 417 μl (3 mmol) of triethylamine. The mixture was stirred at room temperature for 24 hours. After completion of the reaction, the chloroform was distilled off under reduced pressure and the residue was diluted with ethyl acetate and washed successively twice with a 5% aqueous citric acid solution, water, a 5% aqueous sodium hydrogen carbonate solution, and with water, and a saturated aqueous sodium chloride solution in a separatory funnel. The organic layer was dried over anhydrous sodium sulfate.

The sodium sulfate was then filtered off and the filtrate was dried in vacuo to provide 483 mg (yield 44%) of compound (3-1).

12-2: Synthesis of methyl 4-(4-aminobutrylamino)cinnamate hydrochloride [compound (3a-1), k=3, $R^{1a}$=H]

To 409 mg (1.13 mmol) of compound (3-1) was added 4 ml of 4N-HCl/dioxane solution under ice-cooling, and the mixture was stirred at room temperature for 30 minutes. After completion of the reaction, 30 ml of anhydrous ether was added and the resulting precipitate was filtered using a glass filter and washed 2 or 3 times with ether. The resulting solid was dried in vacuo to provide compound (3a-1) as white solid. Yield 92%, m.p. 206.0-208.0°C.

$^1$H-NMR(400MHz, $D_2O$) δ(ppm)=2.07 (2H, quant, $H_2NCH_2\underline{CH_2}CH_2CO$-), 2.62 (2H, t, $H_2NCH_2CH_2\underline{CH_2}CO$-), 3.10 (2H, t, $H_2N\underline{CH_2}CH_2CH_2CO$-), 3.82 (3H, s, $-COOCH_3$), 6.53 (1H, m, -CH=$\underline{CH}$CO-), 7.53 (2H, m, Aromatic H, 2,6 positions), 7.58-7.76 (3H, m, Aromatic H, 3,5 positions, -$\underline{CH}$=CHCO-)

EXAMPLE 13

Synthesis of Methyl 4-(6-Aminohexanoylamino)cinnamate Hydrochloride [compound (3a-2), k=5, $R^{1a}$=H]

13-1: Synthesis of [compound (3-2), k=5, $R^{1a}$=Boc]

The procedure of Example 12-1 was generally repeated to synthesize the title compound. Yield 40%.

13-2: Synthesis of methyl 4-(6-aminohexanoylamino)cinnamate hydrochloride [compound (3a-2), k=5, $R^{1a}$=H]

The title compound was synthesized following essentially the procedure described in Example 12-2. Yield 97%, m.p. 215.4-219.6°C.

$^1$H-NMR(400MHz $D_2O$) δ(ppm)=1.47 (2H, quant, $H_2NCH_2CH_2\underline{CH_2}CH_2CH_2CO$-), 1.72 (4H, m, $H_2NCH_2\underline{CH_2}CH_2\underline{CH_2}CH_2CO$-), 2.48 (2H, t, $H_2NCH_2CH_2CH_2CH_2\underline{CH_2}CO$-), 3.03 (2H, t, $H_2N\underline{CH_2}CH_2CH_2CH_2CH_2CO$-), 3.82 (3H, s, $-COOCH_3$), 6.53 (1H, m, -CH=$\underline{CH}$CO-), 7.51 (2H, d, Aromatic H, 2,6 positions), 7.61-7.76 (3H, m, Aromatic H, 3,5 positions, -$\underline{CH}$=CHCO-)

EXAMPLE 14

Synthesis of Methyl 4-(12-Aminododecanoylamino)cinnamate [compound (3a-3), k=11, $R^{1a}$=H] Hydrochloride

14-1: Synthesis of [compound (3-3), k-11, $R^{1a}$=Boc]

The title compound was synthesized following essentially the procedure described in Example 12-1. Yield 72%.

14-2: Synthesis of methyl 4-(12-aminododecanoylamino)cinnamate [compound (3a-3), k=11, $R^{1a}$=H] hydrochloride

The title compound wee synthesized following essentially the procedure described in Example 12-2. Yield 94%, m.p. 210.2-217.0°C.

$^1$H-NMR(400MHz, $CDCl_3$-DMSO-$D_2O$) δ(ppm)=1.20-1.38 (14H, m, $H_2NCH_2CH_2(\underline{CH_2})_7CH_2CH_2CO$-), 1.53-1.69 (4H, m, $H_2NCH_2\underline{CH_2}(CH_2)_7\underline{CH_2}CH_2CO$-), 2.34 (2H, t, $H_2N(CH_2)_{10}\underline{CH_2}CO$-), 2.80 (2H, t, $H_2N\underline{CH_2}(CH_2)_{10}CO$-), 3.73 (3H, s, -$COOCH_3$), 6.38 (1H, d, -CH=$\underline{CH}$CO-), 7.52 (2H, d, Aromatic H, 2,6 positions), 7.58 (1H, d, -$\underline{CH}$=CHCO-), 7.68 (2H, m, Aromatic H, 3,5 positions)

[3-2] Examples 15-17: Synthesis of a compound corresponding to formulas (3) and (12) as represented by the formula:

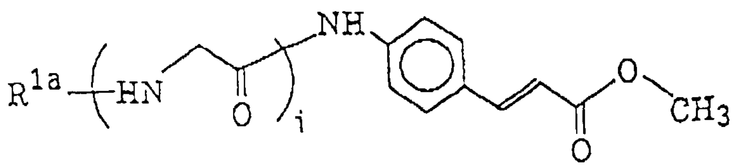

(wherein $R^2$ represents a group of the formula (3), and in the formula (5), $R^3$=$R^4$=H, $R^5$=$CH_3$; and in the formula (12), $R^8$=H)

EXAMPLE 15

Synthesis of Methyl Glycylaminocinnamate [compound (3a-4), i=1, $R^{1a}$=H] Hydrochloride

15-1: Synthesis of [compound (3-4), i=1, $R^{1a}$=Boc]

Using t-butoxycarbonylglycine in lieu of t-butoxycarbonyl-γ-aminobutyric acid, the title compound was synthesized following essentially the procedure described in Example 12-1. Yield 73%.

15-2: Synthesis of methyl glycylaminocinnamate [compound (3a-4), i=1, $R^{1a}$=H] hydrochloride

Using compound (3-4) in lieu of compound (3-1), the title compound (3a-4) was synthesized following essentially the procedure described in Example 12-2. Yield of 98%. m.p. 218.6-225.6°C.

$^1$H-NMR(400MHz, $D_2O$) δ(ppm)=3.86 (3H, s, -$COOCH_3$), 4.02 (2H, s, Gly-α-H), 6.63 (1H, d, -CH=$\underline{CH}$CO-), 7.66 (2H, m, Aromatic H, 2,6 positions), 7.75 (2H, m, Aromatic H, 3,5 positions, -$\underline{CH}$=CHCO-)

EXAMPLE 16

Synthesis of Methyl Glycylglycylaminocinnamate [compound (3a-5), i=2, $R^{1a}$=H] Hydrochloride

16-1: Synthesis of [compound (3-5), i=2, $R^{1a}$=Boc]

In 3 ml of chloroform was dissolved 526 mg (3 mmol) of t-butoxycarbonylglycine and, under ice-cooling, 416 μl (3 mmol) of triethylamine and 1 ml of a solution of 386 mg (3 mmol) dimethylphosphinothioyl chloride in chloroform were added successively. The mixture was stirred at room temperature for 15 minutes, after which 416 μl (3 mmol) of triethylamine and 10 ml of a previously prepared solution of 812 mg (3 mmol) of compound (3a-4) and 416 μl (3 mmol) of triethylamine in chloroform was added under ice-cooling, and the mixture was stirred at room temperature for 20 minutes. This reaction mixture was concentrated under reduced pressure and the residue was diluted with ethyl acetate and washed successively twice with a 5% aqueous citric acid solution, water, a 5% aqueous sodium hydrogen carbonate solution, and with water, and a saturated aqueous sodium chloride solution. The organic layer was dried over anhydrous sodium sulfate. The sodium sulfate was filtered off and the filtrate was concentrated under reduced pressure. The resulting white crystals were washed with ether and dried in vacuo to provide 986 mg (yield 84%) of compound (3-5). Structure was confirmed by the $^1$H-NMR.

$^1$H-NMR(400MHz, $CDCl_3$) δ(ppm)=1.45 (9H, s, Boc-), 3.80 (3H, s, -$COOCH_3$), 3.85 (2H, s, BocGly α-H), 4.15 (2H, s, -Gly$\underline{Gly}$- α-H), 5.15 (1H, br, Boc$\underline{NH}$-), 6.40 (1H, d, -CH=$\underline{CH}$CO-), 7.55 (4H, dd, Aromatic H), 7.65 (1H, d, -$\underline{CH}$=CHCO-), 8.55 (1H, br, Gly$\underline{NH}$Ar-)

16-2: Synthesis of methyl glycylglycylaminocinnamate [compound (3a-5), i=2, $R^{1a}$=H] hydrochloride

To 543 mg (1.4 mmol) of compound (3-5) was added 4 ml of 4M HCl/dioxane under ice-cooling and the mixture was stirred for 40 minutes. To this mixture was added ether and the resulting crystals were filtered and washed with ether. This crystal crop was dried in vacuo to provide 199 mg (yield 61%) of compound (3a-5) as white crystals. m.p. 219.3-231.0°C.

$^1$H-NMR(400MHz, $D_2O$) δ(ppm)=3.83 (3H, s, -$COOCH_3$), 3.94 (2H, s, GlyGly- α-H), 4.19 (2H, s, GlyGly- α-H), 6.54 (1H, d, -CH=CHCO-), 7.54 (2H, d, Aromatic H, 2,6 positions), 7.68 (2H, d, Aromatic H, 3,5 positions), 7.73 (1H, d, -CH=CHCO-)

EXAMPLE 17

Synthesis of Methyl Triglycylaminocinnamate [compound (3a-6), i=3, $R^{1a}$=H] Hydrochloride

17-1: Synthesis of [compound (3-6), i=3, $R^{1a}$=Boc]

In 1 ml of dioxane was dissolved 88 mg (0.5 mmol) of t-butoxycarbonylglycine and, under ice-cooling, 69.5 μl (0.5 mmol) of triethylamine and 1 ml of a solution of 64 mg (0.5 mmol) dimethylphosphinothioyl chloride in dioxane were added successively. The mixture was stirred at room temperature for 25 minutes. Then, 69.5 μl (0.5 mmol) of triethylamine and 1 ml of a previously prepared solution of 163 mg (0.5 mmol) of compound (3a-5) and 69.5 μl (0.5 mmol) of triethylamine in dioxane were added under ice-cooling and the mixture was stirred at room temperature for 1 hour. To this reaction mixture was added 1 ml of aqueous ammonia, followed by stirring for 20 minutes. This solution was concentrated under reduced pressure and the residue was diluted with ethyl acetate and washed successively twice with a 5% aqueous citric acid solution, water, a 5% aqueous sodium hydrogen carbonate solution, and with water, and saturated aqueous sodium chloride solution. The organic layer was dried over anhydrous sodium sulfate. The sodium sulfate was then filtered off and the filtrate was concentrated under reduced pressure. The resulting white crystal crop was washed with ether and dried in vacuo to provide 153 mg (yield 89%) of compound (3-6).

17-2: Synthesis of methyl triglycylaminocinnamate [compound (3a-6), i=3, $R^{1a}$=H] hydrochloride

To 137 mg (0.4 mmol) of compound (3-6) was added 3 ml of 4M HCl/dioxane under ice-cooling and the mixture was stirred for 2 hours. To this reaction mixture was added ether and the resulting crystals were collected by filtration and washed with ether. This crystal crop was dried in vacuo to provide 110 mg (yield 98%) of compound (3a-6) as white crystals. m.p. 227.3-235.8°C.

$^1$H-NMR(400MHz, $D_2O$) δ(ppm)=3.83 (3H, s, -$COOCH_3$), 3.94 (2H, s, GlyGlyGly- α-H), 4.12 (2H, s, GlyGlyGly- α-H), 4.14 (2H, s, GlyGlyGly- α-H), 6.54 (1H, dd, -CH-CHCO-), 7.55 (2H, d, Aromatic H, 2,6 positions), 7.66 (2H, d, Aromatic H, 3,5 positions), 7.72 (1H, dd, -CH=CHCO-)

[Synthesis of cinnamic acid-polymer derivatives and crosslinked cinnamic acid-polymer derivatives]

The cinnamic acid-polymer derivative of this invention prior to UV irradiation is molded in shape of film suitable for UV irradiation (this film is referred to as the photocurable film) and the crosslinked cinnamic acid-polymer after UV irradiation is referred to as the cured film. The degree of gelation mentioned in the examples were calculated by the following equation.

$$\text{Degree of gelation (\%)} = (\text{re-dry weight of film/dry weight of film}) \times 100$$

where

re-dry weight of the film = the weight found by immersing the film in 10,000 parts by volume of water for 24 hours at room temperature, recovering the film by filtration, drying it in vacuo, and weighing the dried film;
dry weight of the film = the weight found by drying the film in vacuo prior to wetting and weighing the dried film.

The degree of cinnamic acid derivative substitution, or DS (degree of substitution), was calculated from the molar ratio of the cinnamic acid derivative introduced per disaccharide unit of hyaluronic acid or chondroitin sulfate

based on NMR or absorbance data by the following equation.

DS (%) = 100 x (the number of moles of cinnamic acid derivative introduced per constituent disaccharide unit)

Examples 18-28

The cinnamic acid-polymer derivatives of formula (13) and the corresponding crosslinked cinnamic acid-polymer derivatives, in which $P^1$ represents hyaluronic acid.

EXAMPLE 18

18-1: Preparation of a photocurable film incorporated with compound (1a-1) [compound (1a-1-HA)]

In 60 ml of water was dissolved 400 mg (1.0 mmol disaccharide unit) of a hyaluronic acid with an average molecular weight of 800,000, followed by addition of 30 ml of 1,4-dioxane. Under ice-cooling, 300 μl of 0.2 M aqueous solution of N-hydroxysuccinimide and 300 μl of 0.1 M aqueous solution of a water-soluble carbodiimide were successively added and, after 5 minutes of stirring, 300 μl of 0.1 M aqueous solution of compound (1a-1) was added. After the mixture was stirred at room temperature for 4 hours, and then 350 ml of saturated sodium acetate/ethanol solution was added so as to precipitate the objective compound. The precipitate was separated by centrifugation (2500 rpm x 5 min.). The sediment was washed three times with 80% ethanol and dissolved in 175 ml of water. The solution was cast in 2 square-well dishes (each, 90 mm x 62 mm) and dried in an oven at 45°C to provide samples of a film. Yield 350 mg. The film had a DS of 0.58% as determined by its absorbance.

18-2: Preparation of a cured film by UV crosslinking of the photocurable film [compound (1a-1-HA)]

The film prepared in Example 18-1 was sandwiched between a pair of 2.4 mm-thick Pyrex glass sheets and irradiated from either side with ultraviolet light using a UV irradiator (light source: a 3 kW metal halide lamp, irradiation distance 125 mm, conveyer speed 1 m/min., wavelength 250-450 nm) for 4 minutes each side or a total of 8 minutes.

Degree of gelation: 107%

EXAMPLES 19-24 and 26-28

Using the cinnamic acid derivatives listed in Table 1 shown below, photocurable films and cured films were prepared in accordance with the procedure of Example 18.

EXAMPLE 25

25-1: Preparation of a photocurable film [compound (1a-8-HA)] incorporated with compound (1a-8)

In 50 ml of water was dissolved 200 mg (0.5 mmol disaccharide unit) of a hyaluronic acid with an average molecular weight of 800,000, followed by addition of 10 ml of 1,4-dioxane. Under ice-cooling, 200 μl of 0.5 M N-hydroxysuccinimide/dioxane solution and 200 μl of 0.25 M water-soluble carbodiimide/aqueous solution were successively added. The mixture was stirred for 5 minutes and 1 ml of an aqueous solution of 14 mg (0.05 mmol) of compound (1a-8) was added. After the mixture was stirred at room temperature for 2 hours, 350 ml of saturated sodium acetate/ethanol solution was added so as to precipitate the objective compound. The precipitate was separated by centrifugation (2500 rpm x 5 min.). The sediment was washed three times with a mixture of water-ethanol and dissolved in 80 ml of water. The solution was cast in a square-well dish (90 mm x 62 mm) and dried in an oven at 45 °C to provide a film. Yield 201 mg. The film had a DS of 0.7% as determined by its absorbance.

25-2: Preparation of a cured film by UV crosslinking of the photocurable film [compound (1a-8-HA)]

The film prepared in Example 25-1 was sandwiched between a pair of 2.4 mm-thick Pyrex glass sheets and irradiated from either side with ultraviolet light using a UV irradiator (light source: a 3 kW metal halide lamp, irradiation

distance 125 mm, conveyer speed 1 m/min., wavelength 250-450 nm) for 2 minutes each side or a total of 4 minutes.
Degree of gelation: 94%

EXAMPLES 29-34

The cinnamic acid-polymer derivatives of formula (15) and the corresponding crosslinked cinnamic acid-polymer derivatives, in which $P^1$ represents hyaluronic acid.

EXAMPLE 29

29-1: Preparation of a photocurable film [compound (3a-1-HA)] incorporated with compound (3a-1)

In 50 ml of water was dissolved 200 mg (0.5 mmol disaccharide unit) of a hyaluronic acid with an average molecular weight of 800,000, followed by addition of 10 ml of 1,4-dioxane. Under ice-cooling, 200 µl of 0.5 M N-hydroxysuccinimide/dioxane solution and 200 µl of 0.25 M aqueous solution of a water-soluble carbodiimide were successively added and, after 2 minutes of stirring, 1 ml of an aqueous solution of 12 mg (0.05 mmol) of compound (3a-1) was added. The mixture was stirred at room temperature for 3 hours and 40 minutes, and then 350 ml of a saturated sodium acetate/ethanol solution was added so as to precipitate the objective compound. The precipitate was separated by centrifugation (2500 rpm x 5 min.). The sediment was washed three times with a mixture of water-ethanol and dissolved in 40 ml of water. The solution was cast in a square-well dish (90 mm x 62 mm) and dried in an oven at 45°C to provide a film. Yield 182 mg. The film had a DS of 1.7% as determined by its absorbance.

29-2: Preparation of a cured film by UV crosslinking of the photocurable film [compound (3a-1-HA)]

The film prepared in Example 29-1 was sandwiched between a pair of 2.4 mm-thick Pyrex glass sheets and irradiated from either side with ultraviolet light using a UV irradiator (light source: a 3 kW metal halide lamp, irradiation distance 125 mm, conveyer speed 1 m/min., wavelength 250-450 nm) for 2 minutes each aide or a total of 4 minutes.
Degree of gelation: 99%

EXAMPLES 30-34

Using the cinnamic acid derivatives listed in Table 1, photocurable films and cured films were prepared in accordance with the procedure of Example 29.

EXAMPLE 35 (Reference Example)

In the preparation of a cinnamic acid-polymer derivative of formula (13) and the corresponding crosslinked cinnamic acid-polymer derivative, chondroitin sulfate was used for $P^1$. The A-$P^1$ linkage is the amide linkage involving the carboxyl group or sulfate group of $P^1$.

35-1: Preparation of a photocurable chondroitin sulfate film [compound (1a-4-CS)] incorporated with compound (1a-4)

Using sodium chondroitin sulfate in lieu of sodium hyaluronate, the title compound was synthesized following essentially the procedure described in Example 25-1. DS=0.79%.

35-2: Preparation of a cured film by UV crosslinking of the photocurable film [compound (1a-4-CS)]

The film prepared in Example 35-1: was sandwiched between a pair of 2.4 mm-thick Pyrex glass sheets and irradiated from either side with ultraviolet light using a UV irradiator (light source: a 3 kW metal halide lamp, irradiation distance 125 mm, conveyer speed-1 m/min., wavelength 250-450 run) for 2 minutes each side or a total of 4 minutes.

EXAMPLES 36-41 (Reference Example)

In the preparation of a cinnamic acid-polymer derivative of formula (15) and the corresponding crosslinked cinnamic acid-polymer derivative, chondroitin sulfate was used for $P^1$. The C-$P^1$ linkage is the amide linkage with the carboxyl group or sulfate group of $P^1$.

EXAMPLE 36 (Reference Example)

36-1: Preparation of a photocurable chondroitin sulfate film [compound (3a-1-CS)] incorporated with compound (3a-1)

Using sodium chondroitin sulfate in lieu of sodium hyaluronate, the title compound was synthesized essentially following the procedure described in Example 29-1. (DS=6%) 36-2: preparation of a cured film by UV crosslinking of the photocurable film [compound (3a-1-CS)]

The film prepared in Example 29-1 was sandwiched between a pair of 2.4 mm-thick Pyrex glass sheets and irradiated from either side with ultraviolet light using a UV irradiator (light source: a 3 kW metal halide lamp, irradiation distance 125 mm, conveyer speed 1 m/min., wavelength 250-450 nm) for 2 minutes each side or a total of 4 minutes.

Degree of gelation: 73%

EXAMPLES 37-41 (Reference Example)

Using the cinnamic acid derivatives listed in Table 1, photocurable films and cured films were prepared in accordance with the procedure of Example 36.

The results obtained in the foregoing Examples 18-41 are summarized in Tables 1 and 2.

Table 1

| | Cinnamic Acid-Polymer Derivative | Cinnamic Acid Derivative | DS (%) | UV Irradiation Time (min.) | Degree of Gelation (%) |
|---|---|---|---|---|---|
| Example 18 | 1a-1-HA | 1a-1 | 0.58 | 8 | 107 |
| Example 19 | 1a-2-HA | 1a-2 | 0.45 | 8 | 82 |
| Example 20 | 1a-3-HA | 1a-3 | 0.46 | 8 | 95 |
| Example 21 | 1a-4-HA | 1a-4 | 0.37 | 8 | 92 |
| Example 22 | 1a-5-HA | 1a-5 | 0.36 | 8 | 97 |
| Example 23 | 1a-6-HA | 1a-6 | 0.25 | 8 | 91 |
| Example 24 | 1a-7-HA | 1a-7 | 0.39 | 8 | 99 |
| Example 25 | 1a-8-HA | 1a-8 | 0.7 | 4 | 94 |
| Example 26 | 1a-9-HA | 1a-9 | 0.99 | 8 | - |
| Example 27 | 1a-10-HA | 1a-10 | 1.0 | 8 | - |
| Example 28 | 1a-11-HA | 1a-11 | 0.7 | 8 | - |

Table 2

| | Cinnamic Acid-Polymer Derivative | Cinnamic Acid Derivative | DS (%) | UV Irradiation Time (min.) | Degree pf Gelation (%) |
|---|---|---|---|---|---|
| Example 29 | 3a-1-HA | 3a-1 | 1.0 | 4 | 95 |
| Example 30 | 3a-2-HA | 3a-2 | 0.4 | 4 | 92 |
| Example 31 | 3a-3-HA | 3a-3 | 0.3 | 4 | 94 |
| Example 32 | 3a-4-HA | 3a-4 | 1.7 | 4 | 99 |
| Example 33 | 3a-5-HA | 3a-5 | 1.8 | 4 | 97 |
| Example 34 | 3a-6-HA | 3a-6 | 1.3 | 4 | 99 |
| Example 35 | 1a-4-CS | 1a-4 | 0.79 | 4 | - |
| Example 36 | 3a-1-CS | 3a-1 | 6 | 4 | 73 |
| Example 37 | 3a-2-CS | 3a-2 | 7 | 4 | 48 |
| Example 38 | 3a-3-CS | 3a-3 | 6 | 4 | 67 |

Table 2 (continued)

| | Cinnamic Acid-Polymer Derivative | Cinnamic Acid Derivative | DS (%) | UV Irradiation Time (min.) | Degree pf Gelation (%) |
|---|---|---|---|---|---|
| Example 39 | 3a-4-CS | 3a-4 | 7 | 4 | 76 |
| Example 40 | 3a-5-CS | 3a-5 | 8 | 4 | 65 |
| Example 41 | 3a-6-CS | 3a-6 | 7 | 4 | 75 |

Crosslinking group-dependent characteristics of photocured hyaluronic acid films

In the following example, differences in film characteristics due to differences in the value of n in the structural formula of the hyaluronic acid derivative incorporated wiht a cinnamic acid derivative of the structural formula $H_2N-(CH_2)n-OCOCH=CH-Ph$ (1-A) (Ph represents phenyl) were mainly investigated.

EXAMPLE 42

In this example, differences in the reactivity to hyaluronic acid in the introduction of the cinnamic acid derivative according to differences in the value of n in the above compound (1-A) under otherwise the identical conditions were investigated (Fig. 2). In Fig. 2, the abscissa represents the amount of cinnamic acid derivative used per hyaluronic acid disaccharide unit under the following conditions and the ordinate represents the degree of substitution DS.

[Reaction conditions]

Host compound: sodium hyaluronate (MW 800,000)
Solvent: water-dioxane (2:1)
Condensing agent: N-hydroxysuccinimide, 2 molar equivalents/(1-A)
Water-soluble carbodiimide, equimolar equivalent/(1-A)
Reaction temperature: room temperature
Reaction time: 24 hrs.

Fig. 2 shows that the longer the methylene chain is, that is to say the larger the value of n is, the lower is the reactivity. Thus, in the reaction in the aqueous solvent, a larger value of n results in an increase, though slight, in the hydrophobicity of compound (1-A) itself and a commensurate decrease in affinity for the solvent or substrate, lowering the reactivity.

EXAMPLE 43

In this example, differences in water absorption rate due to differences in the DS of the crosslinked hyaluronic acid film as exposed to UV light for 4 minutes were investigated introducing a variety of cinnamic acid derivatives varying in the value of n in the formula (1-A) (Fig. 3). The water absorption rate is calculated by the following equation.

Water absorption rate (%) = (wet weight of film - dry weight of film) / dry weight of film x 100

Dry weight of film: weight after drying of cured film
Wet weight of film: weight after 1-hour immersing in water (wetting) of cured film

As DS is decreased, all cured films show steep increases in water absorption rate at below of a specific level of DS. The reason is as follows. A decrease in DS results in a decrease in crosslinking density, rendering the network structure more coarse and enabling to absorb more water. However, a further decrease in crosslink density detracts from the insolubilizability of the film. Analysis of responses due to differences in n reveals that the larger the value of

n is, the greater is the graph shift to downfield on the DS scale. This means that a larger value of n provides a network structure which is able to hold more water and can be insolubilized at a lower value of DS. While construction of a network structure is implemented by photocrosslinking reaction through UV light irradiation, the above results suggest that, when the value of DS being held constant, the photoreactive hyaluronic acid derivative containing a cinnamic acid derivative with a larger value of n is easy to form a network structure, and thus indicate that the photoreactivity of the cinnamic acid derivative has a significant influence on characteristics of the cured film.

EXAMPLE 44

In this example, the responses of tensile strength according to differences in DS of the crosslinked hyaluronic acid films prepared by introducing a variety of cinnamic acid derivatives varying in the value of n in the compound (1-A) and exposing films with UV light for 4 minutes were inestigated (Fig. 4). The tensile strength was invariably measured for the water-immersed film using a rheometer.

It is apparent from Fig. 4 that the larger the value of DS is, the higher is the crosslinking density and, hence, the higher is the tensile strength. Analysis of responses according to differences in the value of n shows that the larger the value of n is, the greater is the graph shift to downfield on the DS scale, indicating that when the value of DS being held constant, a larger value of n provides higher tensile strength.

As described in Example 43 for the same DS, a larger value of n results in a higher photoreaction efficiency to provide a film of higher crosslinking density. Since tensile strength is considered to be proportional to crosslinking density, a larger value of n provides a higher tensile strength.

EXAMPLE 45

In this example, the responses of tensile strength due to differences in the water absorption rate of the crosslinked hyaluronic acid films obtained by introducing a variety of cinnamic acid derivatives varying in the value of n in said compound (1-A) and exposing films with UV light for 4 minutes were investigated (Figs. 5 and 6).

Fig. 5 was constructed by plotting water absorption data on the abscissa and tensile strength data on the ordinate. It is clear that regardless of the value of n, the water absorption rate is inversely proportional to the tensile strength. Fig. 6 was constructed by plotting the reciprocals of tensile strength data on the ordinate, where a linear relation was obtained. 46

EXAMPLE 46

In this example, the critical point of insolubilization of the crosslinking hyaluronic acid films obtained by introducing a variety of cinnamic acid derivatives varying in the value of n in the compound (1-A) and exposing films to UV light for 8 minutes was observed (Fig. 7). The abscissa represents n and the ordinate represents DS. Each ■ plot on the graph represents DS at which the film is solubilized and each + plot represents DS at which insolubilization of the film begins to occur, with the critical point of insolubilization lying between ■ and +. This critical point of insolubiliation is also the point of place transition from film to gel form.

EXAMPLE 47

In this example, the effect of cinnamic acid derivatives other than the compound (1-A), namely the derivatives having structural formula represented by formulas (7) and (9), was investigated.

The relative reactivity of the following crosslinkable compounds to sodium hyaluronate (MW 800,000) was investigated as in Example 42. These cinnamic acid derivatives shown below have an equivalent number of atoms in the backbone chain between the spacer amino group and the cinnamoyl group, but are different in spacer structure, e.g. presence of an ether linkage, an amide linkage or a branch chain in addition to the methylene chain.

$H_2N\text{-}CH_2CH_2CH_2CH_2CH_2\text{-}OCOCH{=}CH\text{-}Ph$ [compound (1a-3)]

$H_2N\text{-}CH_2CH_2\text{-}O\text{-}CH_2CH_2\text{-}OCOCH{=}CH\text{-}Ph$ [compound (1a-7)]

$H_2N\text{-}CH_2\text{-}CONH\text{-}CH_2CH_2\text{-}OCOCH{=}CH\text{-}Ph$ [compound (1a-9)]

$H_2N\text{-}CH(CH_3)\text{-}CONH\text{-}CH_2CH_2\text{-}OCOCH{=}CH\text{-}Ph$ [compound (1a-10)]

$H_2N\text{-}CH[CH_2CH(CH_3)_2]\text{-}CONH\text{-}CH_2CH_2\text{-}OCOCH{=}CH\text{-}Ph$ [compound (1a-11)]

The result is shown in Fig. 8, where the abscissa represents the charged amount of each cinnamic acid derivative per hyaluronic acid disaccharide unit and the ordinate represents the degree of substitution (DS).

The compounds having an amide linkage in the spacer are particularly reactive and, among them, less branched compounds (1a-9) and (1a-10) exhibited better reactivity. This is considered to be a reflection of the effect of hydrophilicity of the spacer structure.

EXAMPLE 48

For the five compounds described in Example 47, the relationship between DS and water absorption rate was investigated by the procedure described in Example 42.

The result is shown in Fig. 9. The hydrophilic compounds having an amide linkage and a lesser degree of branching in spacer structure, (1a-9-HA) and (1a-10-HA), show higher absorption rates at the high DS level as compared with the other compounds. The water absorption rate is markedly influenced by crosslinking density and those compounds with high water absorption rates are lower in crosslinking density than the other compounds. While the crosslinked structure is constructed by photoreaction as described in Example 43, the hyaluronic acid derivative containing the above compound having a highly hydrophilic spacer structure appears to be less reactive for this photoreaction, whereas the derivative with a highly hydrophobic structure shows higher photoreactivity.

While this photoreaction is carried out by casting an aqueous solution of the photoreactive hyaluronic acid derivative into a film and irradiating the film with ultraviolet light, it appears that a cinnamic acid derivative having greater hydrophobicity in film formation tends to aggregate and take to orientation, thus providing for a better environment for photoreaction.

The intensity of hydrophobicity of the above compounds is in the order of compound (1a-3) > compound (1a-7) > compound (1a-11) > compound (1a-10) > compound (1a-9).

The photoreactivity in photoreaction increases in the above order of hydrophobicity and the reactivity to the hyaluronic acid of Example 51 conversely increases in the decreasing order of hydrophobicity.

EXAMPLES 49-52

Enzymatic degradability

A photocurable film prepared according to Example 21 was irradiated with UV light under the conditions shown in Table 2 and the area swelling ratio, degree of gelation, and course of enzymatic degradation (decomposition rate) of the cured film were evaluated (Table 3).

The conditions of enzymatic degradation were as follows.

Buffer: 0.2 M acetic acid-sodium hydroxide (pH 6)
Enzyme: Hyaluronidase (sheep testis origin), 100 U/mg.
Temperature: 37°C
Assay method: The amount of low molecular weight hyaluronic acid released from the cured film by enzymatic degradation was measured by the carbazole method and the decomposition rate was calculated by comparison with the initial weight.

Decomposition rate (%) = (weight of hyaluronic acid

released into buffer/initial weight of cured film) x 100

Table 3

| Example | DS | UV Irradiation (min.) | Area Swelling Ratio (x100%) | Degree of Gelation (%) | Time Course of Enzymatic Degradation (decomposition rate, %) | | | | | |
|---|---|---|---|---|---|---|---|---|---|---|
| | | | | | 0.5H | 3H | 6H | 1D | 2D | 3D |
| 49 | 1.5 | 4 | 2.23 | 96 | 17 | 70 | 88 | 96 | 93 | 92 |
| 50 | 1.5 | 8 | 1.96 | 99 | 10 | 63 | 87 | 93 | 93 | 89 |
| 51 | 1.5 | 12 | 1.99 | 100 | 10 | 50 | 66 | 76 | 75 | 80 |
| 52 | 0.6 | 8 | 3.24 | 93 | 46 | 89 | 89 | 89 | 89 | 93 |
| H: hour; D: day. | | | | | | | | | | |

As described above, this invention of a cinnamic acid derivative containing a spacer enables the synthesis of cinnamic acid-polymer derivatives, which have high sensitivity and high efficiency of photoreaction and, hence, the provision of crosslinked cinnamic acid-hyaluronic acid derivatives retaining the inherent beneficial properties of the hyaluronic acid such as bioresorption, biocompatibility, no toxicity, no antigenicity, and high water absorption.

## Claims

**1.** A cinnamic acid derivative represented by any of the formula (1) or (3) or a salt thereof;

$$R^1\text{-A-H} \quad (1)$$

$$R^2\text{-C-H} \quad (3)$$

wherein $R^1$ represents a group of the formula (4):

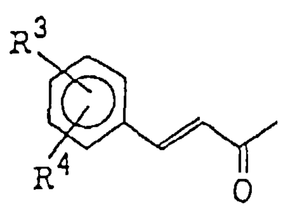

(4)

wherein $R^3$ and $R^4$ independently represent a hydrogen atom, a nitro group, an amino group, a hydroxy group or a $C_{1-4}$ alkoxy group;
$R^2$ represents a group of the formula (5):

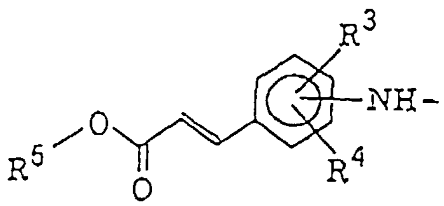

(5)

wherein $R^3$ and $R^4$ are as defined above; $R^5$ represents a $C_{1-4}$ alkyl group;
A-H represents an intramolecularly amino- and hydroxy-containing compound residue represented by any of the

formulas (6)-(9):

$$-O-(CH_2)n-NH_2 \quad (6)$$

wherein n represents a whole number of 3-18,

$$-(O-CH_2CH_2)m-NH_2 \quad (7)$$

wherein m represents a whole number of 2-10,

$$-O-CHR^6CH(COOR^7)-NH_2 \quad (8)$$

wherein $R^6$ represents a hydrogen atom or a $C_{1-4}$ alkyl group; $R^7$ represents a $C_{1-4}$ alkyl group,

$$-O-(CH_2)\ell-NHCO-CHR^8-NH_2 \quad (9)$$

wherein $\ell$ represents a whole number of 2-18; $R^8$ represents the side chain of an $\alpha$-amino acid residue;
C-H represents an amino acid residue of the formula (11) or (12):

$$-CO-(CH_2)k-NH_2 \quad (11)$$

wherein k is representing a whole number of 1-18,

$$-(COCHR^8NH)i-H \quad (12)$$

wherein i represents a whole number of 1-6; $R^8$ is as defined above under the proviso that $R^1$-A-H does not represent O-cinnamoyl-DL-serine methyl ester hydrobromide and $R^2$-C-H does not represent 4-benzyloxy-L-phenylalanine 4-(2-methoxycarbonylvinyl)anilide hydrochloride or 3-phenoxy-DL-phenylalanine 4-(2-methoxycarbonylvinyl) anilide hydrochloride.

**2.** A cinnamic acid-polymer derivative represented by any of the formulas (13) or (15).

$$R^1-A-P^1 \quad (13)$$

$$R^2-C-P^1 \quad (15)$$

wherein $R^1$ represents a group of the formula (4):

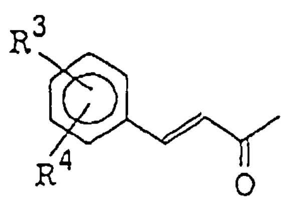

(4)

wherein $R^3$ and $R^4$ independently represent a hydrogen atom, a nitro group, an amino group, a hydroxy group or a $C_{1-4}$ alkoxy group;
$R^2$ represents a group of the formula (5)t

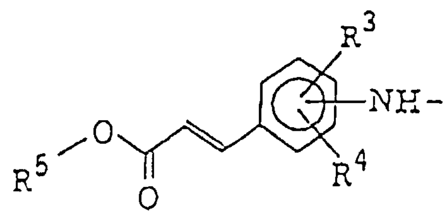 (5)

wherein $R^3$ and $R^4$ are as defined above; $R^5$ represents a $C_{1-4}$ alkyl group;
A- represents an amino- and hydroxy-containing compound residue represented by any of the formulas (6')-(9'):

$$-O-(CH_2)n-NH \quad (6')$$

wherein n represents a whole number of 3-18,

$$-(O-CH_2CH_2)m-NH \quad (7')$$

wherein m represents a whole number of 2-10,

$$-O-CHR^6CH(COOR^7)-NH \quad (8')$$

wherein $R^6$ represents a hydrogen atom or a $C_{1-4}$ alkyl group;
$R^7$ represents a $C_{1-4}$ alkyl group,

$$-O-(CH_2)\ell-NHCO-CHR^8-NH \quad (9')$$

wherein $\ell$ represents a whole number of 2-18; $R^8$ represents the side chain of an $\alpha$-amino acid residue;
C- represents an amino acid residue of the formula (11') or (12'):

$$-CO-(CH_2)k-NH \quad (11')$$

wherein k is representing a whole number of 1-18,

$$-(COCHR^8NH)i \quad (12')$$

wherein i represents a whole number of 1-6; $R^8$ is as defined above;
$P^1$ represents a hyaluronic acid;
the A-$P^1$ linkage is the amide bond formed between the terminal amino group of the formula (6'), (7'), (8') or (9') and the carboxyl group of $P^1$;
and the C-$P^1$ linkage is the amide bond formed between the terminal amino group of the formula (11') or (12') and the carboxyl group of $P^1$,
wherein the degree of substitution of the cinnamic acid derivative is 0.05-5.0% per disaccharide unit of the hyaluronic acid , and wherein said hyaluronic acid has a number average molecular weight of 100,0005,000,000.

**3.** A crosslinked cinnamic acid-polymer derivative produced by photodimerization of $R^1$ and $R^1$, $R^2$ and $R^2$, or $R^1$ and $R^2$ of the cinnamic acid-polymer derivative claimed in Claim 2 to form a crosslinked cyclobutane ring.

**4.** The crosslinked cinnamic acid-polymer derivative of Claim 3 , which is a water-insoluble crosslinked hyaluronic acid derivative in the form of film, gel or powder.

**5.** A crosslinked cinnamic acid-hyaluronic acid derivative wherein the cinnamic acid derivative is one or more members selected from the group consisting of the formulas (1) or (3) or a salt thereof:

$$R^1\text{-A-H} \quad (1)$$

$$R^2\text{-C-H} \quad (3)$$

wherein $R^1$ represents a group of the formula (4):

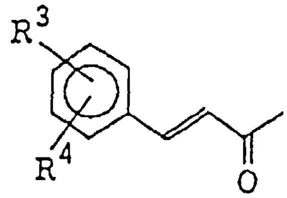

(4)

wherein $R^3$ and $R^4$ independently represent a hydrogen atom, a nitro group, an amino group, a hydroxy group or a $C_{1-4}$ alkoxy group;
$R^2$ represents a group of the formula (5):

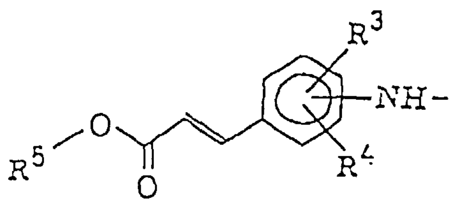

(5)

wherein $R^3$ and $R^4$ are as defined above; $R^5$ represents a $C_{1-4}$ alkyl group;
A-H represents an amino- and hydroxy-containing compound residue represented by any of the formulas (6)-(9):

$$\text{-O-(CH}_2\text{)n-NH}_2 \quad (6)$$

wherein n represents a whole number of 3-18,

$$\text{-(O-CH}_2\text{CH}_2\text{)m-NH}_2 \quad (7)$$

wherein m represents a whole number of 2-10,

$$\text{-O-CHR}^6\text{CH(COOR}^7\text{)-NH}_2 \quad (8)$$

wherein $R^6$ represents a hydrogen atom or a $C_{1-4}$ alkyl group; $R^7$ represents a $C_{1-4}$ alkyl group,

$$\text{-O-(CH}_2\text{)}\ell\text{-NHCO-CHR}^8\text{-NH}_2 \quad (9)$$

wherein $\ell$ represents a whole number of 2-18; $R^8$ represents the side chain of an $\alpha$-amino acid residue;
C-H represents an amino acid residue of the formula (11) or (12):

$$-CO-(CH_2)k-NH_2 \quad (11)$$

wherein k is representing a whole number of 1-18,

$$-(COCHR^8NH)i-H \quad (12)$$

wherein i represents a whole number of 1-6; $R^8$ is as defined above;
wherein the formula (1) or (3) is bonded to the carboxyl group of hyaluronic acid via the amide bond and $R^1$ and $R^1$, $R^2$ and $R^2$, or $R^1$ and $R^2$ of the cinnamic acid-polymer derivative is photodimerized to form a crosslinked cyclobutane ring, wherein the degree of substitution of the cinnamic acid derivative is 0.05-5.0% per disaccharide unit of the hyaluronic acid , and wherein said hyaluronic acid has a number average molecular weight of 100,000-5,000,000.

**6.** A process for producing the cinnamic acid-hyaluronic acid derivative wherein the degree of substitution of the cinnamic acid derivative is 0.05 - 5.0 % per disaccharide unit of the hyaluronic acid and wherein said hyaluronic acid has a number average molecular weight of 100,000 - 5,000,000 which comprises dissolving the hyaluronic acid in water or in water containing a water-miscible organic solvent and reacting the carboxyl group of the hyaluronic acid with the amino group of the cinnamic acid derivative represented by the formula (1) or (3):

$$R^1-A-H \quad (1)$$

$$R^2-C-H \quad (3)$$

wherein $R^1$ represents a group of the formula (4):

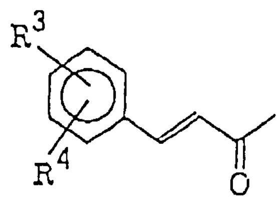
(4)

wherein $R^3$ and $R^4$ independently represent a hydrogen atom, a nitro group, an amino group, a hydroxy group or a $C_{1-4}$ alkoxy group;
$R^2$ represents a group of the formula (5):

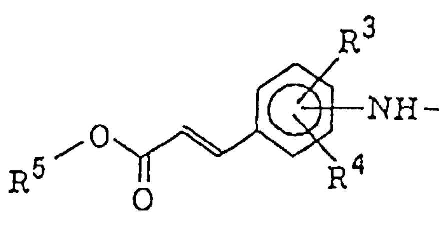
(3)

wherein $R^3$ and $R^4$ are as defined above; $R^5$ represents a $C_{1-4}$ alkyl group;
A-H represents an amino- and hydroxy-containing compound residue represented by any of the formulas (6)-(9):

$$-O-(CH_2)n-NH_2 \quad (6)$$

wherein n represents a whole number of 3-18,

$$-(O\text{-}CH_2CH_2)m\text{-}NH_2 \qquad (7)$$

wherein m represents a whole number of 2-10,

$$-O\text{-}CHR^6CH(COOR^7)\text{-}NH_2 \qquad (8)$$

wherein $R^6$ represents a hydrogen atom or a $C_{1-4}$ alkyl group; $R^7$ represents a $C_{1-4}$ alkyl group,

$$-O\text{-}(CH_2)\ell\text{-}NHCO\text{-}CHR^8\text{-}NH_2 \qquad (9)$$

wherein $\ell$ represents a whole number of 2-18; $R^8$ represents the side chain of an $\alpha$-amino acid residue;
C-H represents an amino acid residue of the formula (11) or (12):

$$-CO\text{-}(CH_2)k\text{-}NH_2 \qquad (11)$$

wherein k is representing a whole number of 1-18,

$$-(COCHR^8NH)i\text{-}H \qquad (12)$$

wherein i represents a whole number of 1-6; $R^8$ is as defined above,
in the presence of a water-soluble carbodiimide and an auxiliary condensing agent.

**7.** A process for producing the crosslinked cinnamic acid-hyaluronic acid derivative of claim 3 which comprises dissolving the derivative obtained by the process of Claim 6 in water or a water-miscible organic solvent, removing the water or water-miscible organic solvent from the resulting solution, forming the photodimerizable hyaluronic acid derivative into a shaped article and irradiating the shaped article with ultraviolet light.

**Patentansprüche**

**1.** Zimtsäurederivat, dargestellt durch eine der Formeln (1) oder (3), oder ein Salz hiervon:

$$R^1\text{-}A\text{-}H \qquad (1)$$

$$R^2\text{-}C\text{-}H \qquad (3)$$

wobei $R^1$ eine Gruppe der Formel (4) darstellt:

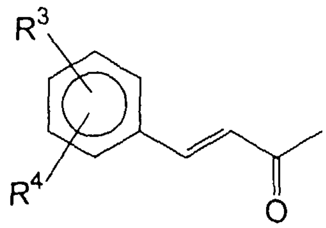

(4)

wobei $R^3$ und $R^4$ unabhängig voneinander ein Wasserstoffatom, eine Nitrogruppe, eine Aminogruppe, eine Hydroxygruppe oder eine $C_{1-4}$-Alkoxygruppe darstellen;

$R^2$ eine Gruppe der Formel (5) darstellt:

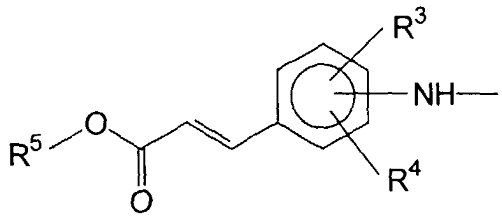

(5)

wobei $R^3$ und $R^4$ wie oben definiert sind; und $R^5$ eine $C_{1-4}$-Alkylgruppe darstellt;

A-H stellt einen Rest einer intramolekular amino- und hydroxyhaltigen Verbindung dar, dargestellt durch eine der Formeln (6) bis (9):

$$-O-(CH_2)_n-NH_2 \quad (6)$$

wobei n eine ganze Zahl von 3 bis 18 darstellt,

$$-(O-CH_2CH_2)_m-NH_2 \quad (7)$$

wobei m eine ganze Zahl von 2 bis 10 darstellt

$$-O-CHR^6CH(COOR^7)-NH_2 \quad (8)$$

wobei $R^6$ ein Wasserstoffatom oder eine $C_{1-4}$-Alkylgruppe darstellt; und $R^7$ eine $C_{1-4}$-Alkylgruppe darstellt,

$$-O-(CH_2)_\ell-NHCO-CHR^8-NH_2 \quad (9)$$

wobei $\ell$ eine ganze Zahl von 2 bis 18 darstellt; und $R^8$ die Seitenkette eines $\alpha$-Aminosäurerestes darstellt;

C-H stellt einen Aminosäurerest der Formel (11) oder (12) dar:

$$-CO-(CH_2)_k-NH_2 \quad (11)$$

wobei k eine ganze Zahl von 1 bis 18 darstellt,

$$-(COCHR^8NH)_i-H \quad (12)$$

wobei i eine ganze Zahl von 1 bis 6 darstellt; $R^8$ wie oben definiert ist, unter der Voraussetzung, dass $R^1$-A-H nicht O-Cinnamoyl-DL-serinmethylester-hydrobromid darstellt und $R^2$-C-H nicht 4-Benzyloxy-L-phenylalanin-4-(2-methoxycarbonylvinyl)anilid-hydrochlorid oder 3-Phenoxy-DL-phenylalanin-4-(2-methoxycarbonylvinyl)anilid-hydrochlorid darstellt.

**2.** Zimtsäurepolymerderivat einer der Formeln (13) oder (15):

$$R^1-A-P^1 \quad (13)$$

$$R^2\text{-C-P}^1 \qquad (15)$$

wobei $R^1$ eine Gruppe der Formel (4) darstellt:

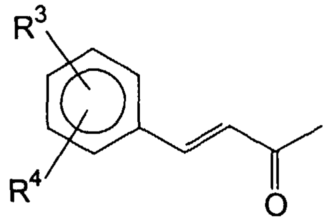

(4)

wobei $R^3$ und $R^4$ unabhängig voneinander ein Wasserstoffatom, eine Nitrogruppe, eine Aminogruppe, eine Hydroxygruppe oder eine $C_{1-4}$-Alkoxygruppe darstellen;

$R^2$ eine Gruppe der Formel (5) darstellt:

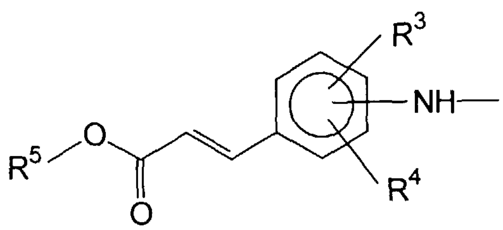

(5)

wobei $R^3$ und $R^4$ wie oben definiert sind; und $R^5$ eine $C_{1-4}$-Alkylgruppe darstellt;

A- einen Rest einer amino- und hydroxyhaltigen Verbindung darstellt, dargestellt durch eine der Formeln (6') bis (9'):

$$\text{-O-(CH}_2)_n\text{-NH} \qquad (6')$$

wobei n eine ganze Zahl von 3 bis 18 darstellt,

$$\text{-(O-CH}_2\text{CH}_2)_m\text{-NH} \qquad (7')$$

wobei m eine ganze Zahl von 2 bis 10 darstellt,

$$\text{-O-CHR}^6\text{CH(COOR}^7\text{)-NH} \qquad (8')$$

wobei $R^6$ ein Wasserstoffatom oder eine $C_{1-4}$-Alkylgruppe darstellt; $R^7$ eine $C_{1-4}$-Alkylgruppe darstellt;

$$\text{-O-(CH}_2)_\ell\text{-NHCO-CHR}^8\text{-NH} \qquad (9')$$

wobei $\ell$ eine ganze Zahl von 2 bis 18 darstellt; $R^8$ die Seitenkette eines Aminosäurerestes darstellt;

C- stellt einen Aminosäurerest der Formel (11') oder (12') dar:

$$\text{-CO-(CH}_2)_k\text{-NH} \qquad (11')$$

wobei k eine ganze Zahl von 1 bis 18 darstellt,

$$-(COCHR^8NH)_i \qquad (12')$$

wobei i eine ganze Zahl von 1 bis 6 darstellt; und $R^8$ wie oben definiert ist;
$P^1$ stellt Hyaluronsäure dar;

die A-$P^1$-Verbindung ist die Amidbindung, die zwischen der terminalen Aminogruppe der Formel (6'), (7'), (8') oder (9') und der Carboxygruppe von $P^1$ gebildet wird;
und die C-$P^1$-Verbindung ist die Amidbindung, die zwischen der terminalen Aminogruppe der Formel (11') oder (12') und der Carboxygruppe von $P^1$ gebildet wird,

wobei der Substitutionsgrad des Zimtsäurederivats 0,05 bis 5,0 % pro Disaccharideinheit der Hyaluronsäure mit einem zahlengemittelten Molekulargewicht von 100.000 bis 5.000.000 beträgt.

**3.** Vernetztes Zimtsäurepolymerderivat, hergestellt durch Fotopolymerisation von $R^1$ und $R^1$, $R^2$ und $R^2$, oder $R^1$ und $R^2$ eines Zimtsäurepolymerderivats gemäss Anspruch 2, wobei ein vernetzter Cyclobutanring gebildet wird.

**4.** Vernetztes Zimtsäurepolymerderivat gemäss Anspruch 3, welches ein wasserunlösliches vernetztes Hyaluronsäurederivat in Form eines Filmes, Geles oder Pulvers ist.

**5.** Vernetztes Zimtsäurehyaluronsäurederivat, wobei das Zimtsäurederivat ein oder mehrere Vertreter, ausgewählt aus der Gruppe bestehend aus den Formeln (1) oder (3), oder ein Salz hiervon ist:

$$R^1\text{-A-H} \qquad (1)$$

$$R^2\text{-C-H} \qquad (3)$$

wobei $R^1$ eine Gruppe der Formel (4) darstellt:

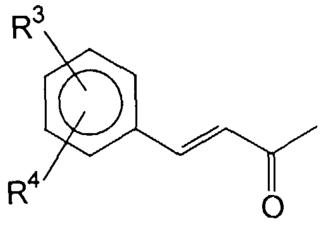

(4)

wobei $R^3$ und $R^4$ unabhängig voneinander ein Wasserstoffatom, eine Nitrogruppe, eine Aminogruppe, eine Hydroxygruppe oder eine $C_{1-4}$-Alkoxygruppe darstellen;
$R^2$ eine Gruppe der Formel (5) darstellt:

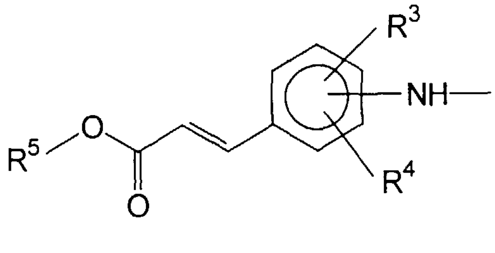

(5)

wobei $R^3$ und $R^4$ wie oben definiert sind; und $R^5$ eine $C_{1-4}$-Alkylgruppe darstellt;

A-H stellt einen Rest einer intramolekular amino- und hydroxyhaltigen Verbindung dar, dargestellt durch eine der Formeln (6) bis (9):

$$-O-(CH_2)_n-NH_2 \quad (6)$$

wobei n eine ganze Zahl von 3 bis 18 darstellt,

$$-(O-CH_2CH_2)_m-NH_2 \quad (7)$$

wobei m eine ganze Zahl von 2 bis 10 darstellt

$$-O-CHR^6CH(COOR^7)-NH_2 \quad (8)$$

wobei $R^6$ ein Wasserstoffatom oder eine $C_{1-4}$-Alkylgruppe darstellt; und $R^7$ eine $C_{1-4}$-Alkylgruppe darstellt,

$$-O-(CH_2)_\ell-NHCO-CHR^8-NH_2 \quad (9)$$

wobei $\ell$ eine ganze Zahl von 2 bis 18 darstellt; und $R^8$ die Seitenkette eines $\alpha$-Aminosäurerestes darstellt; C-H stellt einen Aminosäurerest der Formel (11) oder (12) dar:

$$-CO-(CH_2)_k-NH_2 \quad (11)$$

wobei k eine ganze Zahl von 1 bis 18 darstellt,

$$-(COCHR^8NH)_i-H \quad (12)$$

wobei i eine ganze Zahl von 1 bis 6 darstellt; $R^8$ wie oben definiert ist,

wobei die Formel (1) oder (3) an die Carboxygruppe der Hyaluronsäure über eine Amidbindung gebunden ist, und $R^1$ und $R^1$, $R^2$ und $R^2$, oder $R^1$ und $R^2$ des Zimtsäurepolymerderivats fotodimerisiert ist, wobei ein vernetzter Cyclobutanring gebildet wird, wobei der Substitutionsgrad des Zimtsäurederivats 0,05 bis 5,0 % pro Disaccharideinheit der Hyaluronsäure beträgt, und wobei die Hyaluronsäure ein zahlengemitteltes Molekulargewicht von 100.000 bis 5.000.000 aufweist.

**6.** Verfahren zur Herstellung des Zimtsäurehyaluronsäurederivats, wobei der Substitutionsgrad des Zimtsäurederivats 0,05 bis 5,0 % pro Disaccharideinheit der Hyaluronsäure beträgt, und wobei die Hyaluronsäure ein zahlengemitteltes Molekulargewicht von 100.000 bis 5.000.000 aufweist, welches das Lösen der Hyaluronsäure in Wasser oder in Wasser, enthaltend ein wassermischbares organisches Lösungsmittel, und Umsetzen der Carboxygruppe der Hyaluronsäure mit der Aminogruppe des Zimtsäurederivats, dargestellt durch die Formel (1) oder (3), umfasst:

$$R^1-A-H \quad (1)$$

$$R^2-C-H \quad (3)$$

wobei $R^1$ eine Gruppe der Formel (4) darstellt:

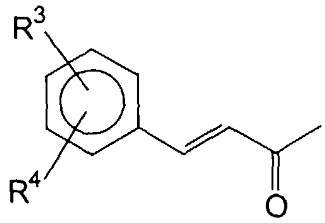

(4)

wobei $R^3$ und $R^4$ unabhängig voneinander ein Wasserstoffatom, eine Nitrogruppe, eine Aminogruppe, eine Hydroxygruppe oder eine $C_{1-4}$-Alkoxygruppe darstellen;

$R^2$ eine Gruppe der Formel (5) darstellt:

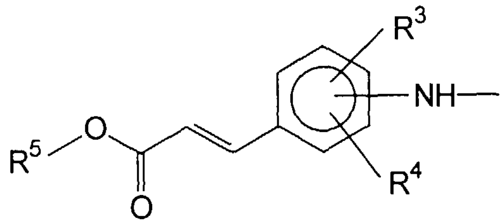

(5)

wobei $R^3$ und $R^4$ wie oben definiert sind; und $R^5$ eine $C_{1-4}$-Alkylgruppe darstellt;

A-H stellt einen Rest einer intramolekular amino- und hydroxyhaltigen Verbindung dar, dargestellt durch eine der Formeln (6) bis (9):

$$-O-(CH_2)_n-NH_2 \quad (6)$$

wobei n eine ganze Zahl von 3 bis 18 darstellt,

$$-(O-CH_2CH_2)_m-NH_2 \quad (7)$$

wobei m eine ganze Zahl von 2 bis 10 darstellt

$$-O-CHR^6CH(COOR^7)-NH_2 \quad (8)$$

wobei $R^6$ ein Wasserstoffatom oder eine $C_{1-4}$-Alkylgruppe darstellt; und $R^7$ eine $C_{1-4}$-Alkylgruppe darstellt,

$$-O-(CH_2)_\ell-NHCO-CHR^8-NH_2 \quad (9)$$

wobei $\ell$ eine ganze Zahl von 2 bis 18 darstellt; und $R^8$ die Seitenkette eines $\alpha$-Aminosäurerestes darstellt;

C-H stellt einen Aminosäurerest der Formel (11) oder (12) dar:

$$-CO-(CH_2)_k-NH_2 \quad (11)$$

wobei k eine ganze Zahl von 1 bis 18 darstellt,

$$-(COCHR^8NH)_i-H \quad (12)$$

wobei i eine ganze Zahl von 1 bis 6 darstellt; $R^8$ wie oben definiert ist,
in Anwesenheit eines wasserlöslichen Carbodiimids und eines Hilfskondensationsmittels.

7. Verfahren zur Herstellung des vernetzten Zimtsäurehyaluronsäurederivats gemäss Anspruch 3, das das Lösen des in dem Verfahren von Anspruch 6 erhaltenen Derivats in Wasser oder einem wassermischbaren organischen Lösungsmittel, das Entfernen des Wassers oder des wassermischbaren organischen Lösungsmittels von der resultierenden Lösung, das Bilden des fotodimerisierbaren Hyaluronsäurederivats in einen geformten Artikel und Bestrahlen des geformten Artikels mit ultraviolettem Licht umfasst.

## Revendications

1. Dérivé d'acide cinnamique représenté par l'une quelconque des formules (1) ou (3) ou un sel de celui-ci :

$$R^1\text{-A-H} \quad (1)$$

$$R^2\text{-C-H} \quad (3)$$

formules dans lesquelles $R^1$ représente un groupe de formule (4) :

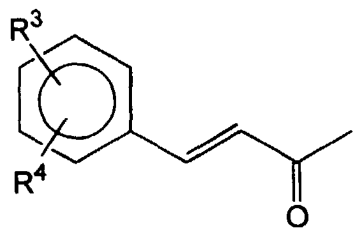

(4)

formule dans laquelle $R^3$ et $R^4$ représentent indépendamment un atome d'hydrogène, un groupe nitro, un groupe amino, un groupe hydroxy ou un groupe alcoxy en $C_1$ à $C_4$ ;
$R^2$ représente un groupe de formule (5) :

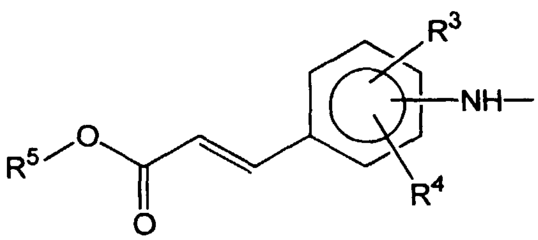

(5)

formule dans laquelle $R^3$ et $R^4$ sont tels que définis ci-dessus ; $R^5$ représente un groupe alkyle en $C_1$ à $C_4$ ;
A-H représente un reste de composé contenant intramoléculairement un groupe amino et hydroxy représenté par l'une quelconque des formules (6) à (9) :

$$-O-(CH_2)_n-NH_2 \quad (6)$$

dans laquelle n représente un nombre entier de 3 à 18,

$$-(O-CH_2CH_2)_m-NH_2 \quad (7)$$

dans laquelle m représente un nombre entier de 2 à 10,

$$-O-CHR^6CH(COOR^7)-NH_2 \quad (8)$$

dans laquelle $R^6$ représente un atome d'hydrogène ou un groupe alkyle en $C_1$ à $C_4$ ; $R^7$ représente un groupe alkyle en $C_1$ à $C_4$,

$$-O-(CH_2)_l-NHCO-CHR^8-NH_2 \quad (9)$$

dans laquelle l représente un nombre entier de 2 à 18 ; $R^8$ représente la chaîne latérale d'un reste d'acide $\alpha$-aminé ; C-H représente un reste d'acide aminé de formule (11) ou (12) :

$$-CO-(CH_2)_k-NH_2 \quad (11)$$

dans laquelle k représente un nombre entier de 1 à 18,

$$-(COCHR^8NH)_i-H \quad (12)$$

dans laquelle i représente un nombre entier de 1 à 6 ; $R^8$ est tel que défini ci-dessus sous réserve que $R^1$-A-H ne représente pas le bromhydrate de l'ester méthylique de la O-cinnamoyl-DL-sérine et que $R^2$-C-H ne représente pas le chlorhydrate de 4-(2-méthoxycarbonylvinyl)anilide de 4-benzyloxy-L-phénylalanine ou le chlorhydrate de 4-(2-méthoxycarbonylvinyl)anilide de 3-phénoxy-DL-phénylalanine.

**2.** Dérivé polymère d'acide cinnamique représenté par l'une quelconque des formules (13) ou (15) :

$$R^1-A-P^1 \quad (13)$$

$$R^2-C-P^1 \quad (15)$$

formules dans lesquelles $R^1$ représente un groupe de formule (4) :

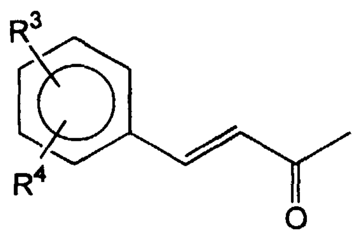

(4)

formule dans laquelle $R^3$ et $R^4$ représentent indépendamment un atome d'hydrogène, un groupe nitro, un groupe amino, un groupe hydroxy ou un groupe alcoxy en $C_1$ à $C_4$ ;
$R^2$ représente un groupe de formule (5) :

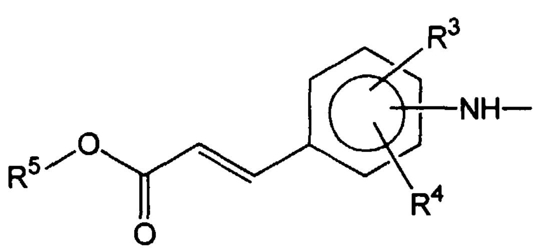

(5)

formule dans laquelle $R^3$ et $R^4$ sont tels que définis ci-dessus ; $R^5$ représente un groupe alkyle en $C_1$ à $C_4$ ; A- représente un reste de composé contenant un groupe amino et hydroxy représenté par l'une quelconque des formules (6') à (9') :

$$-O-(CH_2)_n-NH \quad (6')$$

dans laquelle n représente un nombre entier de 3 à 18,

$$-(O-CH_2CH_2)_m-NH \quad (7')$$

dans laquelle m représente un nombre entier de 2 à 10,

$$-O-CHR^6CH(COOR^7)-NH \quad (8')$$

dans laquelle $R^6$ représente un atome d'hydrogène ou un groupe alkyle en $C_1$ à $C_4$ ; $R^7$ représente un groupe alkyle en $C_1$ à $C_4$,

$$-O-(CH_2)_l-NHCO-CHR^8-NH \quad (9')$$

dans laquelle l représente un nombre entier de 2 à 18 ; $R^8$ représente la chaîne latérale d'un reste d'acide $\alpha$-aminé ; C- représente un reste d'acide aminé de formule (11') ou (12') :

$$-CO-(CH_2)_k-NH \quad (11')$$

dans laquelle k représente un nombre entier de 1 à 18,

$$-(COCHR^8NH)_i \quad (12')$$

dans laquelle i représente un nombre entier de 1 à 6 ; $R^8$ est tel que défini ci-dessus ; $P^1$ représente un acide hyaluronique ; la liaison A-$P^1$ est la liaison amide formée entre le groupe amino terminal de formule (6'), (7'), (8') ou (9') et le groupe carboxyle de $P^1$ ; et la liaison C-$P^1$ est la liaison amide formée entre le groupe amino terminal de la formule (11') ou (12') et le groupe carboxyle de $P^1$, dans lequel le degré de substitution du dérivé d'acide cinnamique est 0,05 à 5,0% par unité disaccharide de l'acide hyaluronique, et dans lequel ledit acide hyaluronique a une masse moléculaire moyenne en nombre de 100 000 à 5 000 000.

3. Dérivé polymère réticulé d'acide cinnamique produit par photodimérisation de $R^1$ et $R^1$, $R^2$ et $R^2$, ou $R^1$ et $R^2$ du dérivé polymère d'acide cinnamique revendiqué dans la revendication 2 pour former un cycle cyclobutane réticulé.

4. Dérivé polymère réticulé d'acide cinnamique de la revendication 3, qui est un dérivé réticulé insoluble dans l'eau d'acide hyaluronique sous forme d'une pellicule, d'un gel ou d'une poudre.

5. Dérivé réticulé d'acide hyaluronique et d'acide cinnamique dans lequel le dérivé d'acide cinnamique est un ou plusieurs membres choisis dans le groupe constitué par les formules (1) ou (3) ou un sel de ceux-ci :

$$R^1-A-H \quad (1)$$

$$R^2-C-H \quad (3)$$

formules dans lesquelles $R^1$ représente un groupe de formule (4) :

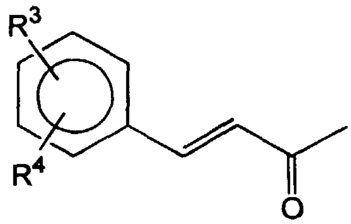
(4)

formule dans laquelle $R^3$ et $R^4$ représentent indépendamment un atome d'hydrogène, un groupe nitro, un groupe amino, un groupe hydroxy ou un groupe alcoxy en $C_1$ à $C_4$ ;
$R^2$ représente un groupe de formule (5) :

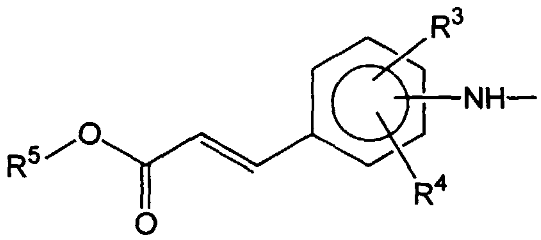
(5)

formule dans laquelle $R^3$ et $R^4$ sont tels que définis ci-dessus ; $R^5$ représente un groupe alkyle en $C_1$ à $C_4$ ;
A-H représente un reste de composé contenant intramoléculairement un groupe amino et hydroxy représenté par l'une quelconque des formules (6) à (9) :

$$-O-(CH_2)_n-NH_2 \quad (6)$$

dans laquelle n représente un nombre entier de 3 à 18,

$$-(O-CH_2CH_2)_m-NH_2 \quad (7)$$

dans laquelle m représente un nombre entier de 2 à 10,

$$-O-CHR^6CH(COOR^7)-NH_2 \quad (8)$$

dans laquelle $R^6$ représente un atome d'hydrogène ou un groupe alkyle en $C_1$ à $C_4$ ; $R^7$ représente un groupe alkyle en $C_1$ à $C_4$,

$$-O-(CH_2)_l-NHCO-CHR^8-NH_2 \quad (9)$$

dans laquelle l représente un nombre entier de 2 à 18 ; $R^8$ représente la chaîne latérale d'un reste d'acide $\alpha$-aminé ;
C-H représente un reste d'acide aminé de formule (11) ou (12) :

$$-CO-(CH_2)_k-NH_2 \quad (11)$$

dans laquelle k représente un nombre entier de 1 à 18,

$$-(COCHR^8NH)_i-H \quad (12)$$

dans laquelle i représente un nombre entier de 1 à 6 ; $R^8$ est tel que défini ci-dessus ; dans lequel la formule (1) ou (3) est liée au groupe carboxyle de l'acide hyaluronique via la liaison amide et $R^1$ et $R^1$, $R^2$ et $R^2$, ou $R^1$ et $R^2$ du dérivé polymère d'acide cinnamique est photodimérisé pour former un cycle cyclobutane réticulé, dans lequel le degré de substitution du dérivé d'acide cinnamique est 0,05 à 5,0% par unité disaccharide de l'acide hyaluronique, et dans lequel ledit acide hyaluronique a une masse moléculaire moyenne en nombre de 100 000 à 5 000 000.

**6.** Procédé de préparation du dérivé d'acide hyaluronique et d'acide cinnamique dans lequel le degré de substitution du dérivé d'acide cinnamique est 0,05 à 5,0% par unité disaccharide de l'acide hyaluronique, et dans lequel ledit acide hyaluronique a une masse moléculaire moyenne en nombre de 100 000 à 5 000 000, qui comprend la dissolution de l'acide hyaluronique dans l'eau ou dans de l'eau contenant un solvant organique miscible à l'eau et la réaction du groupe carboxyle de l'acide hyaluronique avec le groupe amino du dérivé d'acide cinnamique représenté par les formules (1) ou (3) :

$$R^1\text{-A-H} \qquad (1)$$

$$R^2\text{-C-H} \qquad (3)$$

formules dans lesquelles $R^1$ représente un groupe de formule (4) :

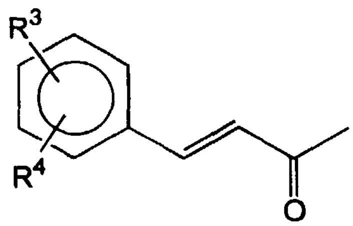

**(4)**

formule dans laquelle $R^3$ et $R^4$ représentent indépendamment un atome d'hydrogène, un groupe nitro, un groupe amino, un groupe hydroxy ou un groupe alcoxy en $C_1$ à $C_4$ ;
$R^2$ représente un groupe de formule (5) :

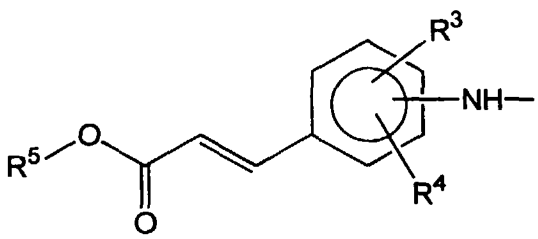

**(5)**

formule dans laquelle $R^3$ et $R^4$ sont tels que définis ci-dessus ; $R^5$ représente un groupe alkyle en $C_1$ à $C_4$ ;
A-H représente un reste de composé contenant un groupe amino et hydroxy représenté par l'une quelconque des formules (6) à (9) :

$$\text{-O-(CH}_2)_n\text{-NH}_2 \qquad (6)$$

dans laquelle n représente un nombre entier de 3 à 18,

$$\text{-(O-CH}_2\text{CH}_2)_m\text{-NH}_2 \qquad (7)$$

dans laquelle m représente un nombre entier de 2 à 10,

$$-O-CHR^6CH(COOR^7)-NH_2 \quad (8)$$

dans laquelle $R^6$ représente un atome d'hydrogène ou un groupe alkyle en $C_1$ à $C_4$ ; $R^7$ représente un groupe alkyle en $C_1$ à $C_4$,

$$-O-(CH_2)_l-NHCO-CHR^8-NH_2 \quad (9)$$

dans laquelle l représente un nombre entier de 2 à 18 ; $R^8$ représente la chaîne latérale d'un reste d'acide $\alpha$-aminé ; C-H représente un reste d'acide aminé de formule (11) ou (12) :

$$-CO-(CH_2)_k-NH_2 \quad (11)$$

dans laquelle k représente un nombre entier de 1 à 18,

$$-(COCHR^8NH)_i-H \quad (12)$$

dans laquelle i représente un nombre entier de 1 à 6 ; $R^8$ est tel que défini ci-dessus ;
en présence d'un carbodiimide soluble dans l'eau et d'un agent de condensation auxiliaire.

7. Procédé de préparation du dérivé d'acide hyaluronique et d'acide cinnamique selon la revendication 3 qui comprend la dissolution du dérivé obtenu par le procédé de la revendication 6 dans l'eau ou un solvant miscible à l'eau, l'élimination de l'eau ou du solvant miscible à l'eau de la solution résultante, la formation du dérivé d'acide hyaluronique photodimérisable en un article en forme et l'irradiation de l'article en forme par la lumière ultraviolette.

Fig. 1

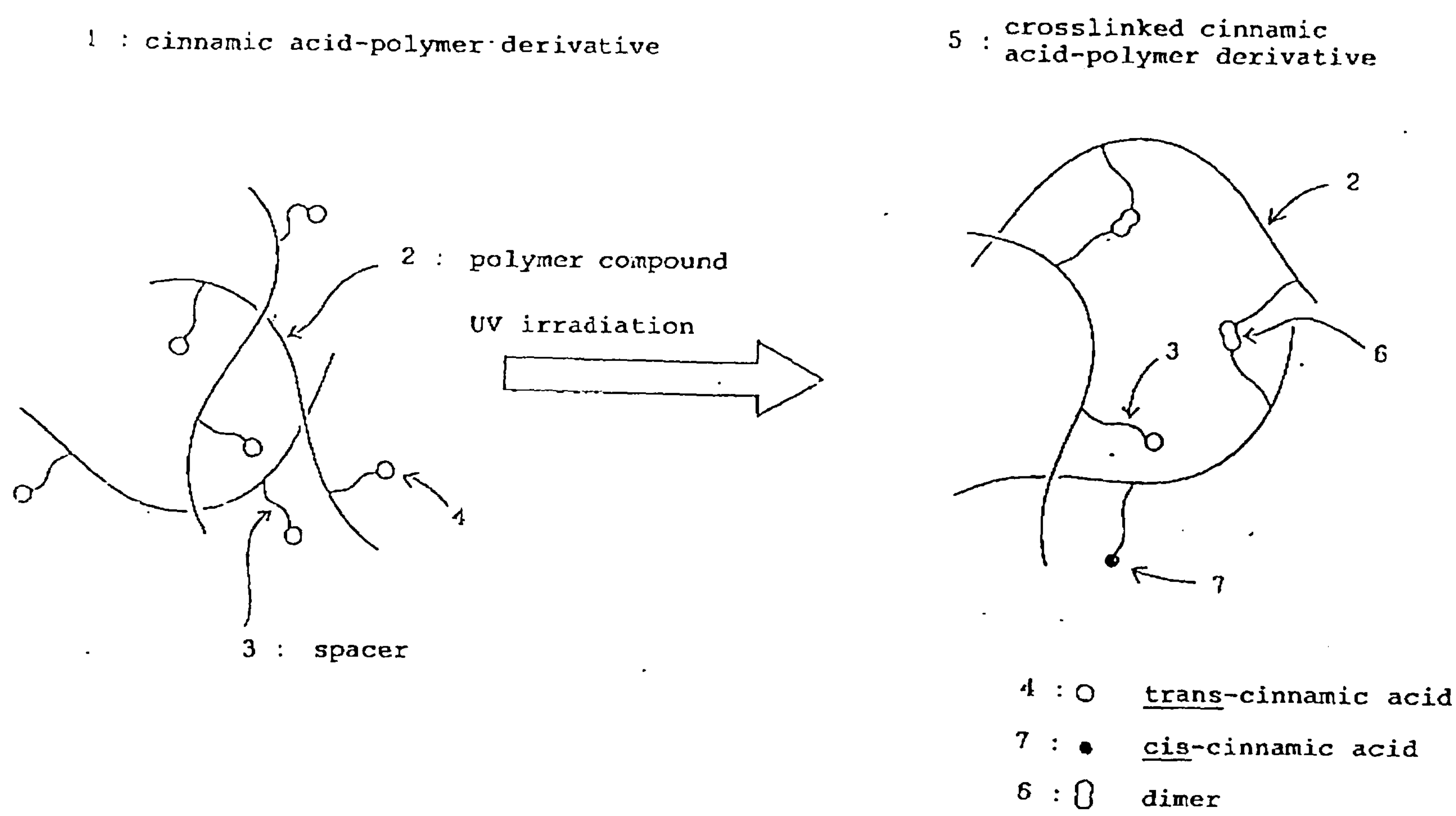
1 : cinnamic acid-polymer·derivative
2 : polymer compound
UV irradiation
3 : spacer
4
5 : crosslinked cinnamic acid-polymer derivative
2
3
6
7
4 : trans-cinnamic acid
7 : cis-cinnamic acid
6 : dimer

Fig. 2

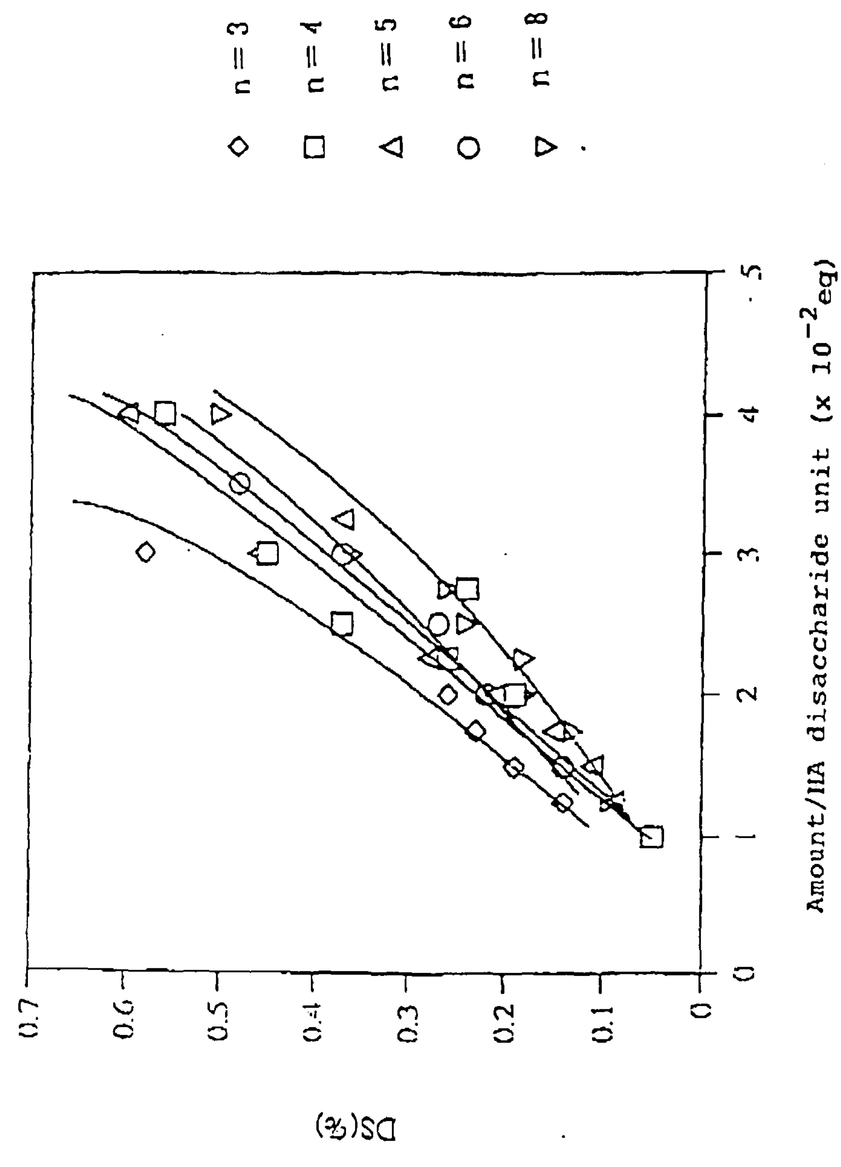
n = 3
n = 4
n = 5
n = 6
n = 8
DS(%)
0.7
0.6
0.5
0.4
0.3
0.2
0.1
0
0
1
2
3
4
5
Amount/HA disaccharide unit (x 10^-2 eq)

Fig. 3

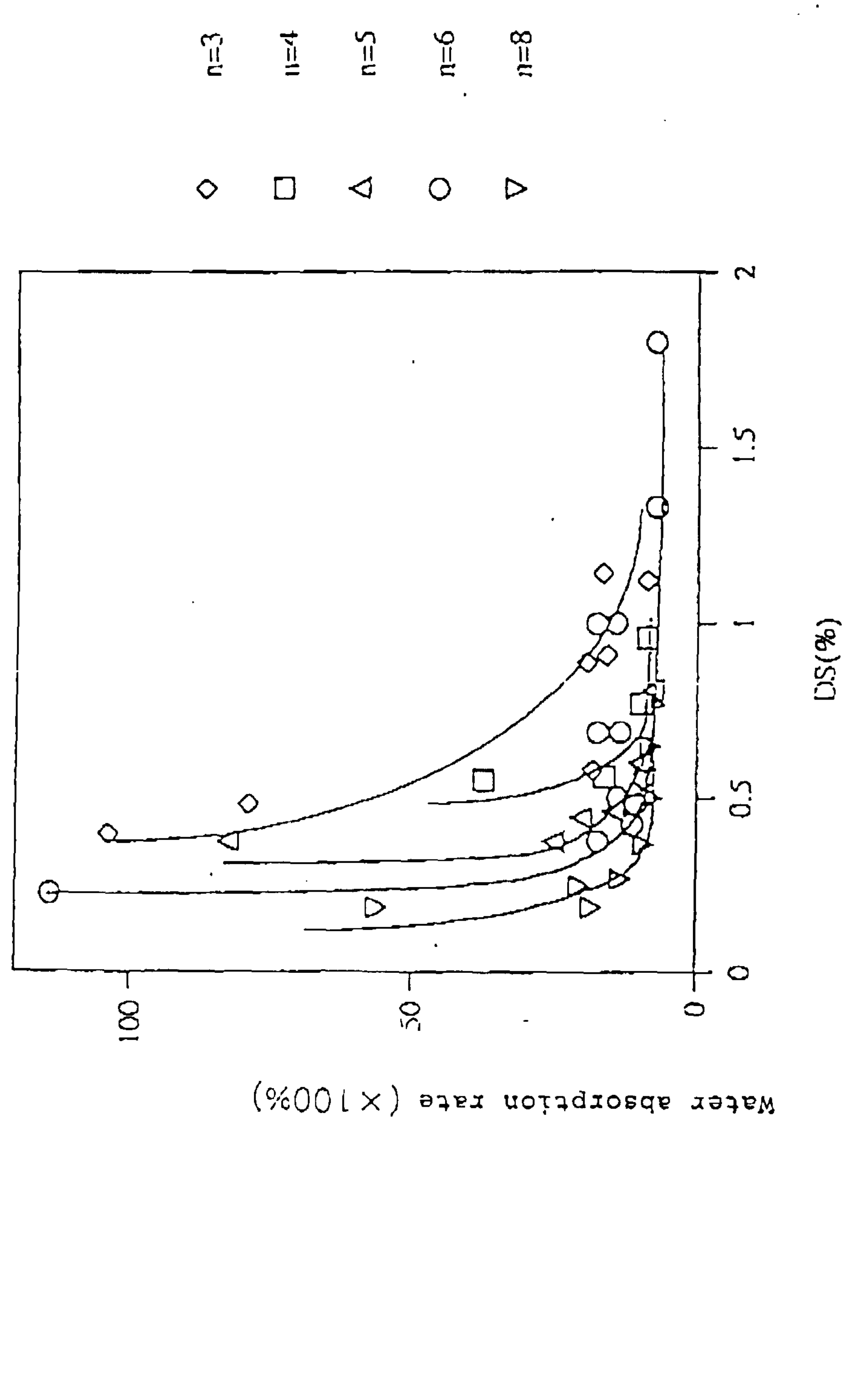

Fig. 4

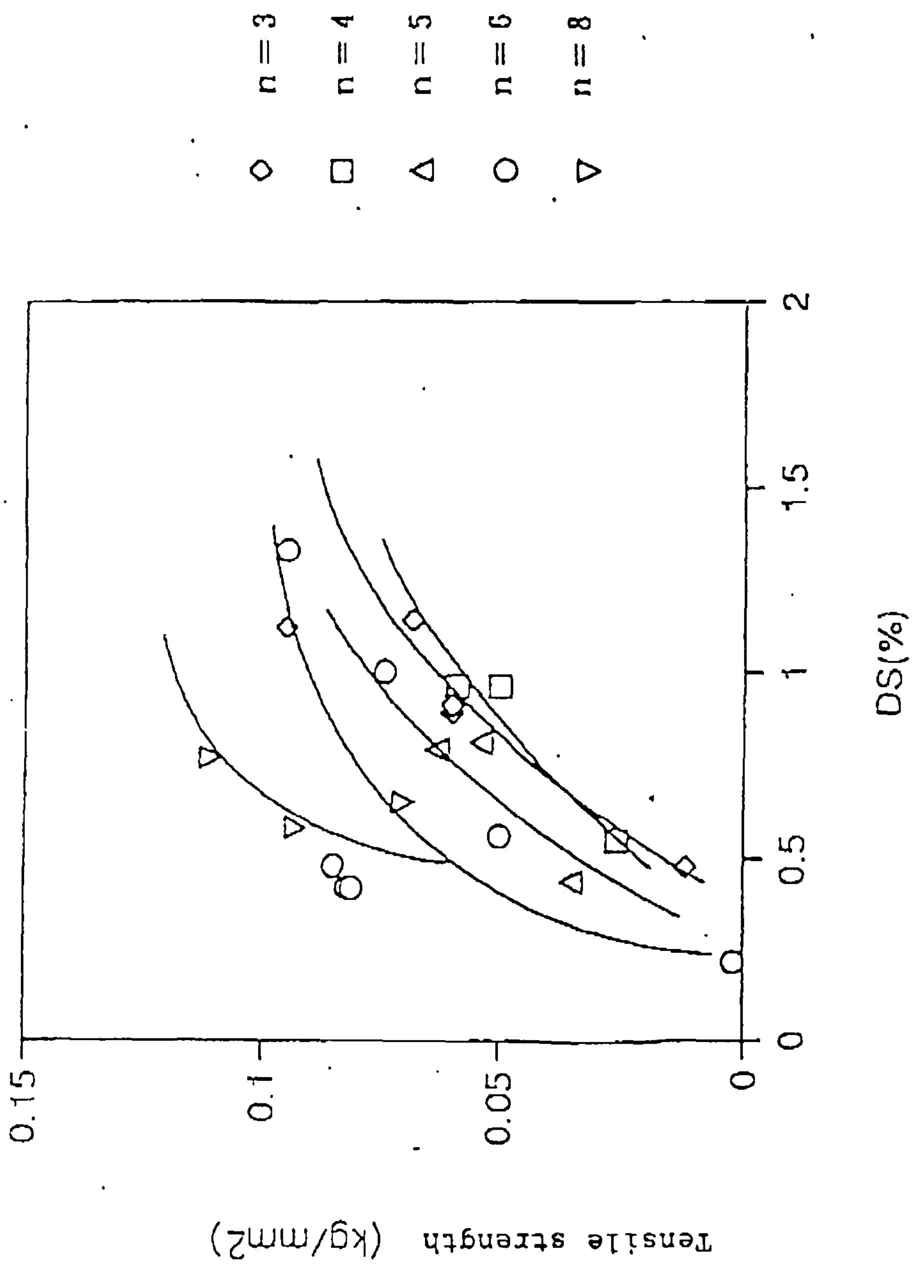

Fig. 5

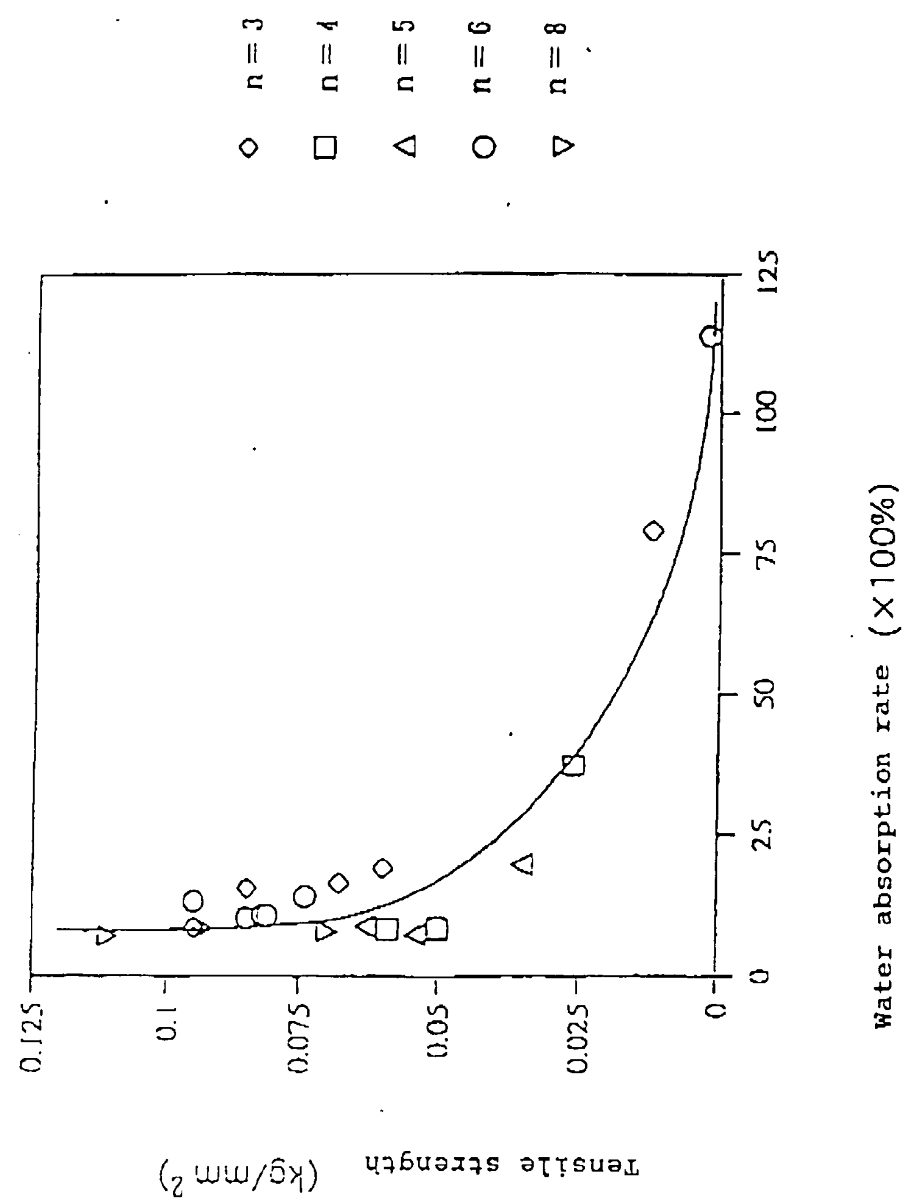
Tensile strength (kg/mm²)
0.125
0.1
0.075
0.05
0.025
0
0
25
50
75
100
125
Water absorption rate (×100%)
◇ n = 3
□ n = 4
△ n = 5
○ n = 6
▽ n = 8

Fig. 6

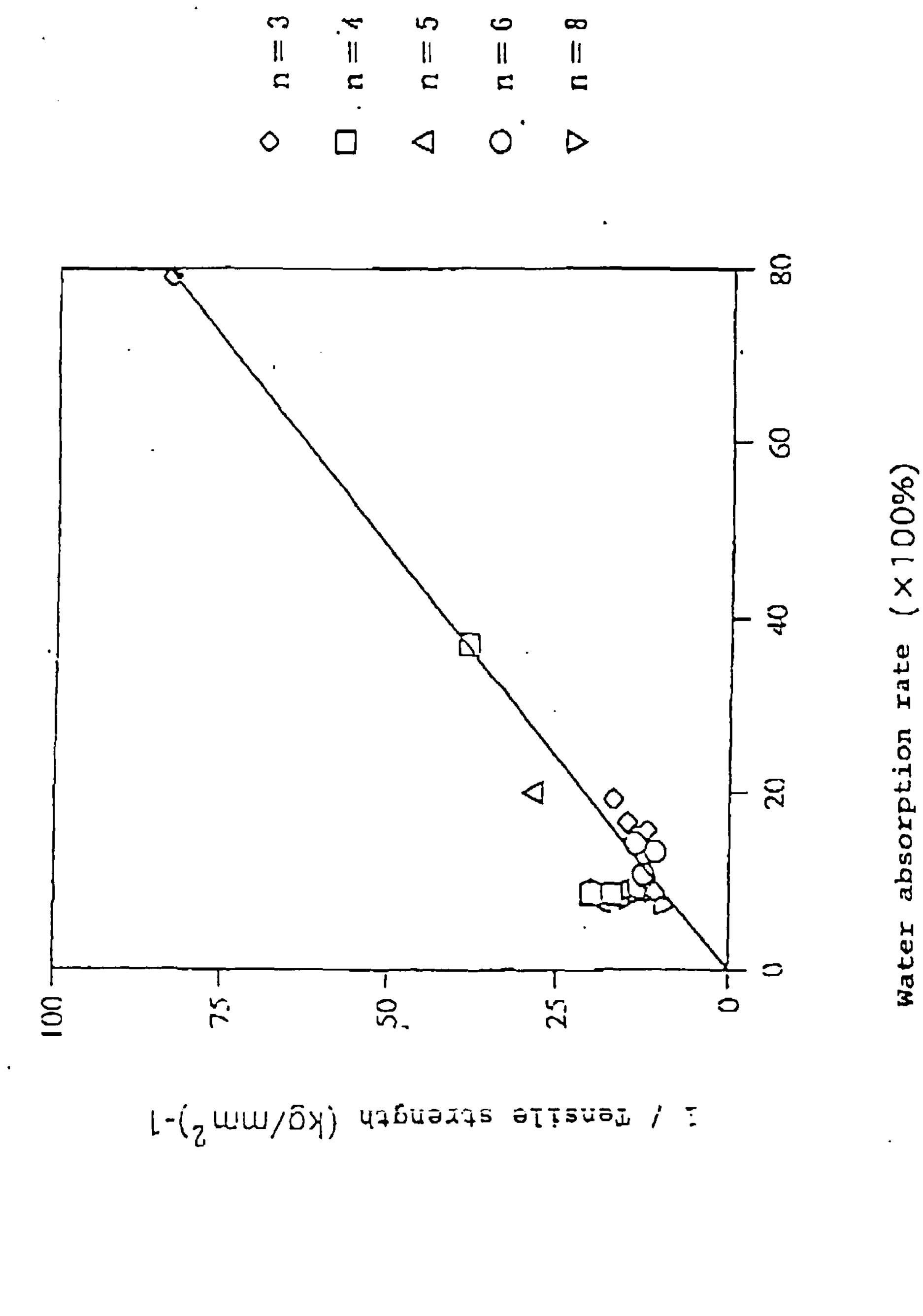
n = 3
n = 4
n = 5
n = 6
n = 8
100
75
50
25
0
0
20
40
60
80
Water absorption rate (×100%)
1 / Tensile strength (kg/mm²)-1

Fig. 7

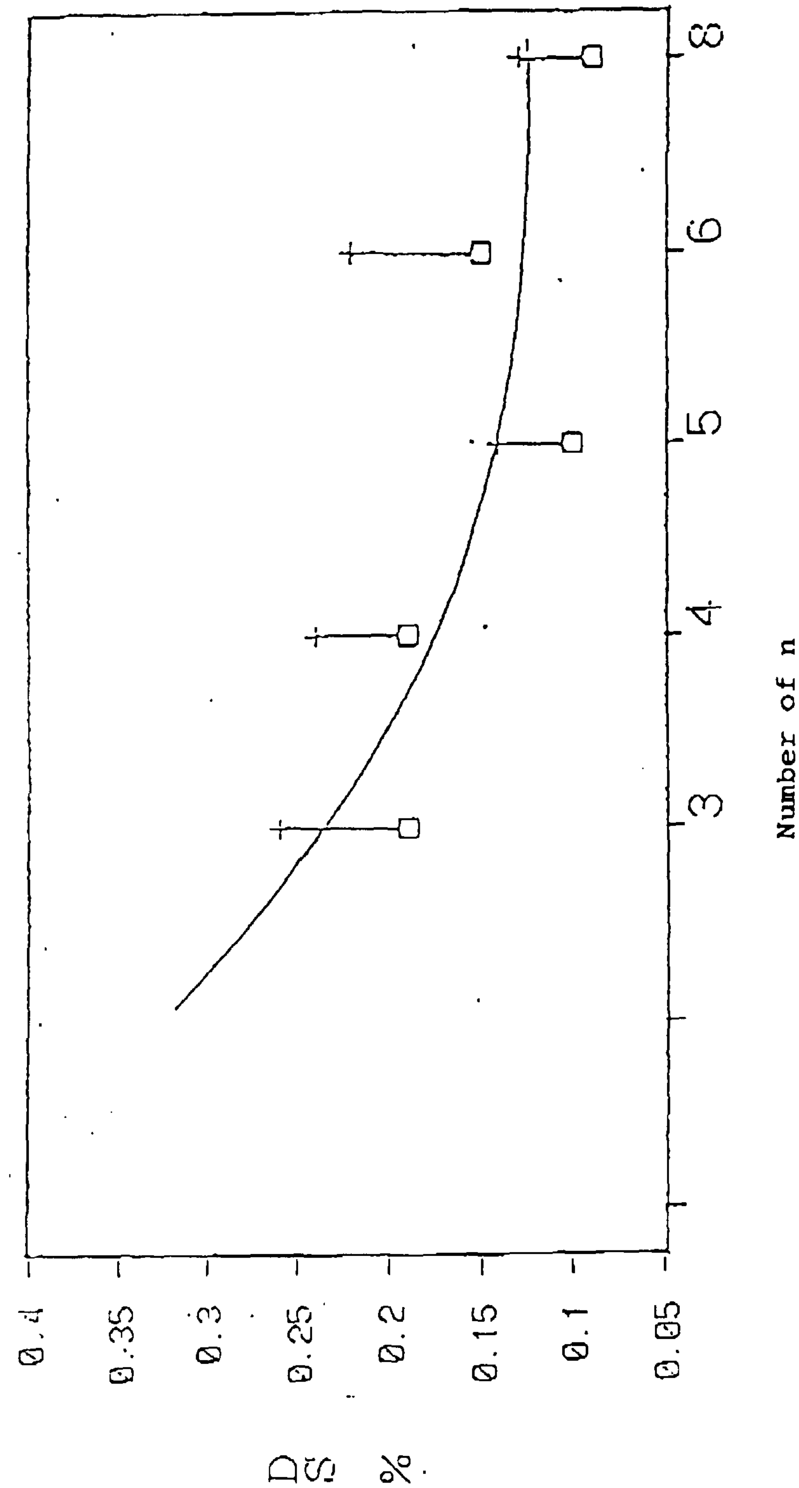

Fig. 8

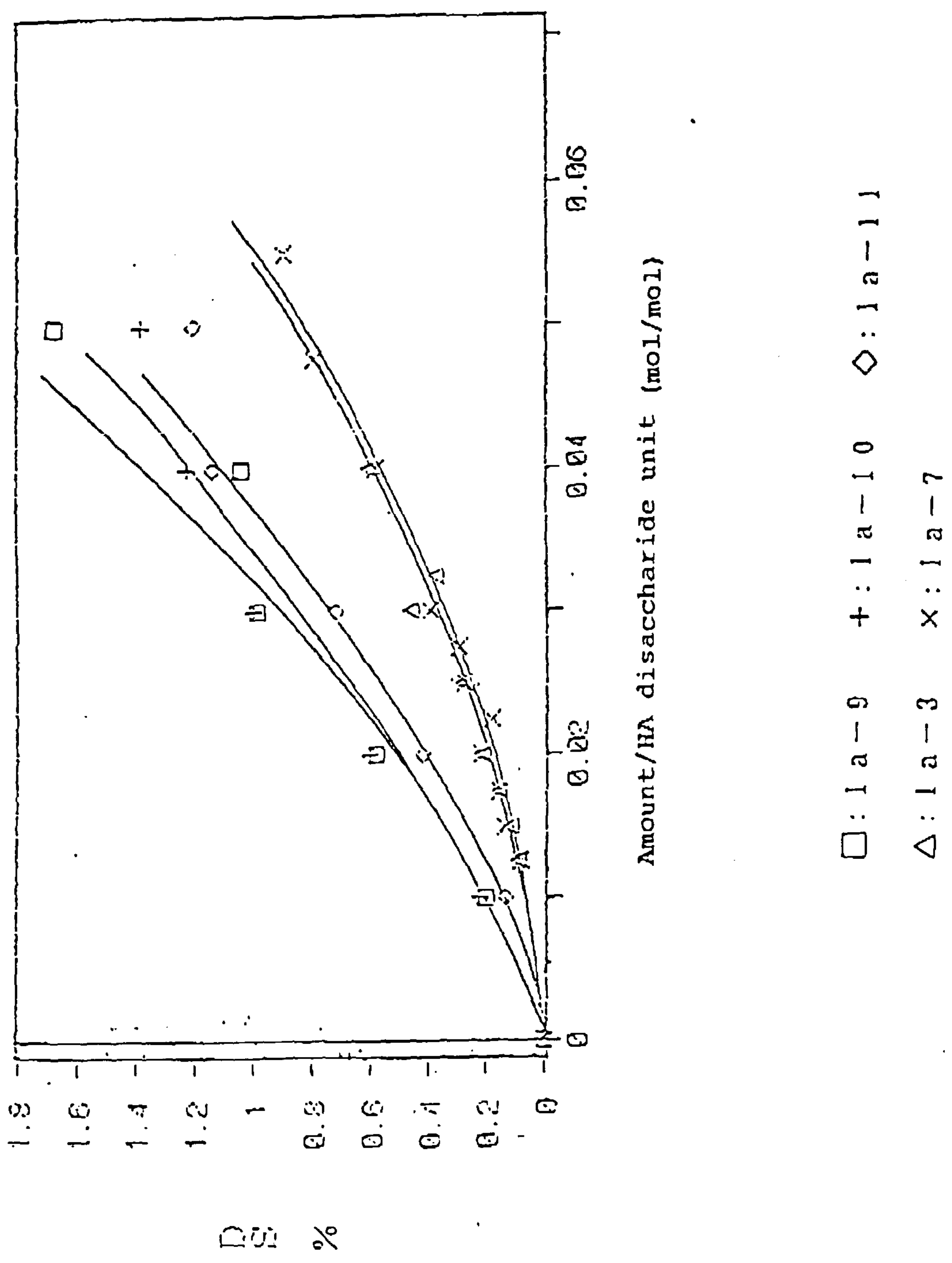

DS %
1.8
1.6
1.4
1.2
1
0.8
0.6
0.4
0.2
0
0
0.02
0.04
0.06
Amount/HA disaccharide unit (mol/mol)
□ : 1 a − 9
+ : 1 a − 1 0
◇ : 1 a − 1 1
△ : 1 a − 3
× : 1 a − 7

Fig. 9

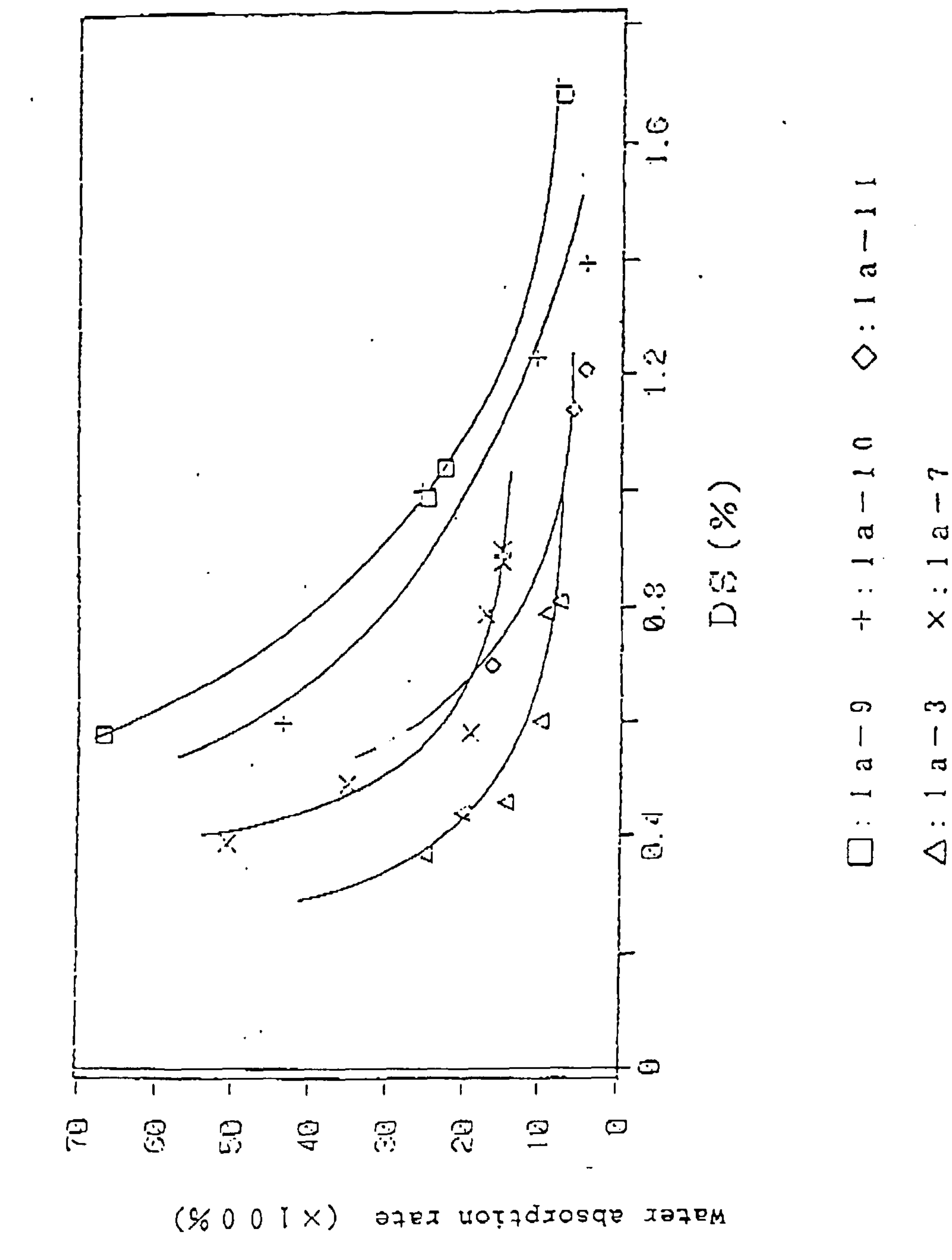

Water absorption rate (×100%)
70
60
50
40
30
20
10
0
0
0.4
0.8
1.2
1.6
DS (%)
□: 1a−9
+: 1a−10
◇: 1a−11
△: 1a−3
×: 1a−7